# EUROPEAN PATENT APPLICATION

(11) **EP 4 796 151 A2**
(43) Date of publication of application: **26.08.2026**
(21) Application number: 26190017.9
(22) Date of filing: 23.10.2023
(51) Int. Cl.: A61M 16/00

(54) **ANAESTHETIC GAS CAPTURE DEVICE**

(30) Priority: 24.10.2022 GB 202215738; 23.12.2022 GB 202219699; 11.05.2023 GB 202307047
(62) Document number(s) of the earlier application(s) in accordance with Art. 76 EPC: 23806037.0 / 4 608 483
(71) Applicant: Sagetech Medical Equipment Limited, Paignton Devon TQ4 7AU (GB)
(72) Inventor: WILEMAN, Steven, Paignton, TQ4 7AU (GB); MURPHY, Christopher, Paignton, TQ4 7AU (GB); BARRET, Darren, Paignton, TQ4 7AU (GB)
(74) Representative: Bryers Intellectual Property Ltd

(57) **Abstract**

An anaesthetic gas capture device for extracting anaesthetic agent from a gas flow, the device comprises or can be connected to suction means for drawing gas flow to the device, the device is configured to receive one or more capture canisters through which gas can flow, the suction means operates upstream of the capture canister/s whereupon the suction means pushes gas through the or one or more of the canisters under positive pressure and agent extraction occurs under positive pressure. The canister includes an inlet and an outlet, a valve is provided for the inlet and the outlet, the inlet and outlet valves being configured to be normally closed to seal the body and configured to open when the canister is loaded into an anaesthetic gas capture device.

## Description

The present invention relates to generally to the capture of halocarbons and more particularly, although not exclusively, to apparatus (systems and/or parts thereof) for use in the capture of anaesthetic agents in medical environments.

Examples of agents for which systems can be configured, adapted or provided include e.g. halogenated ethers (such as isoflurane, desflurane, and sevoflurane), halogenated hydrocarbons (such as halothane), nitrous oxide or xenon.

A halocarbon is an organic chemical molecule composed of at least one carbon atom bound covalently with one or more halogen elements. Halocarbons have many uses and are used in several industries as solvents, pesticides, refrigerants, fire-resistant oils, ingredients of elastomers, adhesives and sealants, electrically insulating coatings, plastics and anaesthetics. An alternative term for halocarbons is "halogenated fluorocarbons" when halogen elements other than fluorine are included in the molecule.

Volatile anaesthetic agents are typically halogenated fluorocarbons, examples of which include desflurane, isoflurane, sevoflurane and halothane. Volatile anaesthetic agents are liquid at room temperature but evaporate easily to produce a vapour for inhalation by a patient to induce anaesthesia. Anaesthetic agents are used extensively in modern healthcare and represent a significant cost. They are also potent greenhouse gases due to their ability to absorb infrared light and their upper atmospheric persistence. Isoflurane and Halothane also contain Chlorine and Bromine groups that contribute to ozone depletion.

Examples of halocarbons which are used as anaesthetic agents typically include desflurane, isoflurane, sevoflurane, halothane and enflurane. These anaesthetics may be referred to as volatile anaesthetic agents because they are liquid at room temperature but evaporate easily to produce a vapour for inhalation by a patient to induce anaesthesia. These agents are administered to patients using the breathing circuit of an ananaesthetic machine, also known as a Boyle's machine. The primary function of the anaesthetic machine is to mix oxygen with volatile anaesthetic agent, at a clinician-specified concentration, for delivery to the patient via the breathing circuit.

The present invention seeks to provide improvements in or relating to the capture of anaesthetic agents from medical environments.

An aspect of the present invention provides an anaesthetic gas capture device for extracting anaesthetic agent from a gas flow, the device comprises or can be connected to suction means for drawing gas flow to the device, the device is configured to receive one or more capture canisters through which gas can flow, the suction means operates upstream of the capture canister/s whereupon the suction means pushes gas through the or one or more of the canisters under positive pressure and agent extraction occurs under positive pressure.

The device may be configured to draw gas from an anaesthetic machine.

The device may be configured to draw from a patient.

The device may be configured to draw from a patient mask.

The device may be pneumatically decoupled from the gas flow source so that the device cannot apply vacuum pressure thereto. An air break may, for example, be provided by the device and/or the source.

The device may comprise a sensor for sensing the presence of anaesthetic agent in gas flow.

The sensor may provided on an inlet side of the device,

The suction means may be provided upstream of the capture canister/s and downstream of the sensor.

In some embodiments the sensor can activate the suction means when anaesthetic agent is detected.

The sensor may activate and/or deactivate the suction means based on detection of a threshold agent concentration or amount.

Devices of some embodiments have a capture mode and a bypass mode.

The device may be automatically switchable between the capture mode and the bypass mode. Alternatively or additionally the device may be manually switchable between the capture mode and the bypass mode.

The device may be connectable to a gas scavenging system.

The device may, for example, be connectable to an Anaesthetic Gas Scavenging System (AGSS).

In some embodiments the device may be configurable to be an AGSS replacement.

The device may, for example, be configured to be pneumatically decoupled from a scavenging system when connected. An air break may, for example, be provided by the device and/or a scavenging system.

The device may be configured such that canisters can be removed/replaced during operation of the device.

Devices may comprise means for determining the remaining capacity of a canister.

Devices may comprise means for detecting the presence of a canister.

The suction means may, for example, be one or more of a vacuum source, a venturi pump or a fan.

In some embodiments, for example, the suction means comprises a plurality of fans.

Some devices comprise or can be associated with one or more air breaks.

Some embodiments comprise an air break formed within or between flow conduits.

Some embodiments comprising a bypass to receive flow when the suction means is inactive and/or to act as a high pressure bypass.

Device may be formed as a mobile trolley.

Devices may be wall mountable.

The present invention also provides a mobile anaesthetic gas scavenging device comprising a device as described herein.

A further aspect provides an anaesthetic gas capture system for extracting anaesthetic gas from the exhaust of an anaesthesia machine, the system comprises an extraction device, the device comprises or can be connected to suction means for drawing gas exhausted from a machine to the device to cause gas to flow through a capture canister, the system comprises an open reservoir from which the suction means draws gas, the machine exhausts into the open reservoir, whereby the device is pneumatically decoupled from the machine so that the device cannot apply vacuum pressure to the machine, the suction means is provided upstream of the capture canister.

The device may be connected to a gas scavenging system. The connection from the device to the scavenging system may include a pneumatic break.

The present invention also provides an anaesthetic gas capture system, the system comprises an anaesthetic agent extraction device, the device comprises or can be connected to suction means for drawing gas including anaesthetic agents thereinto for extraction, the device is pneumatically decoupled from the source of the gas, the suction means is configured so that agent extraction occurs under positive pressure.

The device may be configured to draw gas from one or more of: an anaesthetic machine; a patient; or a patient mask.

The present invention also provides an anaesthetic gas capture device for extracting anaesthetic gas from an anaesthesia machine, the device comprises or is connectable to a suction means for causing gas flow from the machine through the device under positive pressure, the device comprises or can be connected to an air break for pneumatically decoupling the device from the machine so that a generally constant volumetric gas flow is maintained regardless of pneumatic variation caused by the machine or the device.

The present invention also provides an anaesthetic gas capture device for extracting anaesthetic gas from the exhaust of an anaesthesia machine, the device is connected or connectable to an anaesthesia machine, the device comprises or is connectable to a suction means for causing gas flow from the machine through the device, the device comprises or can be connected to an air break for pneumatically decoupling the device from the machine so that a generally constant volumetric gas flow is maintained regardless of pneumatic variation caused by the machine or the device.

The present invention also provides an anaesthetic gas capture device for extracting anaesthetic gas from an anaesthesia machine, comprising suction means for drawing gas exhausted from an anaesthetic machine to the device, the device has means for receiving one or more capture canisters through which exhausted gas can flow, the device comprises an air break such that it cannot apply vacuum pressure to the machine in use, the device comprises a sensor for sensing the presence of anaesthetic agent in gas flow from the machine, the sensor is provided on an inlet side of the device, wherein the suction means is provided upstream of the capture canister/s and downstream of the sensor, in which the sensor can activate the suction means when anaesthetic agent is detected, whereupon the suction means pushes gas through the or one or more of the canisters under positive pressure.

The present invention also provides for a medical environment provided with one or more devices or systems as described herein.

A further aspect of the present invention provides a capture canister for an anaesthetic gas capture machine, the canister comprises a body for receiving filter material and through which gas containing anaesthetic agent can flow, the canister includes an inlet and an outlet, a valve is provided for the inlet and the outlet, the inlet and outlet valves being configured to be normally closed to seal the body and configured to open when the canister is loaded into an anaesthetic gas capture machine.

The inlet valve and/or outlet valve may include a return spring.

The inlet valve and/or outlet valve may be formed as a poppet valve.

The canister may comprises a mesh plate at or towards the inlet and/or outlet.

The mesh plate/s may act as a diffuser.

Part of the valve may be positioned in and/or supported by the mesh.

A further aspect provides an anaesthetic capture device for extracting anaesthetic agent from an anaesthetic machine, the device can releasably receive one or more capture canisters through which gas containing agent can flow.

A further aspect provides a capture device for extracting anaesthetic agent from an anaesthetic environment, the device can releasably receive one or more capture canisters through which gas containing agent can flow.

The device may comprise one or more canister loading mounts for releasably receiving capture canisters.

The device may be configured to push exhausted gas through the or one or more of the canisters under positive pressure.

The device may comprise or may be connected to suction means for drawing gas exhausted from a machine to the device.

The device may be pneumatically decoupled from an anaesthetic machine so that the device cannot apply vacuum pressure to the machine.

The device may comprise a sensor for sensing the presence of anaesthetic agent in gas flow from the machine,

The sensor may be provided on an inlet side of the device,

The suction means may be provided upstream of the capture canister/s and downstream of the sensor.

In some embodiment the sensor can activate the suction means when anaesthetic agent is detected, whereupon the suction means pushes gas through the or one or more of the canisters under positive pressure.

The device may be configured to receive one or more capture canisters as defined herein.

The device may also connected or connectable to a gas scavenging system.

The device may be pneumatically decoupled from the scavenging system.

In some embodiments the or each canister can be removed/replaced during operation of the device ("hot-swap").

The device may comprise means for determining the remaining capacity of a canister.

The device may comprise means for detecting the presence of a canister.

The device/machine/appliance of the present invention may be formed as a mobile trolley.

The device may be wall mountable.

The present invention also provides a mobile anaesthetic gas scavenging device comprising a device as described herein.

A further aspect provides a medical environment provided with one or more devices as described herein.

An aspect of the present invention provides an anaesthetic gas capture device for extracting anaesthetic gas from an anaesthesia machine, the device comprises or can be connected to suction means for drawing gas exhausted from a machine to the device, the device comprises one or more capture canisters through which exhausted gas can flow, the device is pneumatically decoupled from the machine so that the device cannot apply vacuum pressure to the machine, the device comprises a sensor for sensing the presence of anaesthetic agent in gas flow from the machine, the sensor is provided on an inlet side of the device, the suction means is provided upstream of the capture canister/s and downstream of the sensor, in which the sensor can activate the suction means when anaesthetic agent is detected, whereupon the suction means pushes gas through the or one or more of the canisters under positive pressure.

The device may also connected or connectable to a gas scavenging system.

The device may also connected or connectable to an Anaesthetic Gas Scavenging System (AGSS). Some embodiments of the present invention provide an AGSS replacement configuration.

The device may be pneumatically decoupled from the scavenging system.

The device may comprise or may have means for receiving one or more capture canisters through which exhaust gas flows, the canisters contain filter material for capturing anaesthetic agent from the gas flow.

In some embodiments the or each canister can be removed/replaced during operation of the device.

The device may comprise means for determining the remaining capacity of a canister.

The device may comprise means for detecting the presence of a canister.

The suction means may comprise one or more of a vacuum source, a venturi pump or a fan.

The suction means may comprise a plurality of fans.

The device may comprise an air break formed within or between flow conduits.

The device may comprise a bypass to receive flow when the suction means in inactive and/or to act as a high pressure bypass.

The sensor may activate and/or deactivate the suction means based on detection of a threshold agent concentration or amount. For example, the sensor may not activate a fan until a threshold minimum level of agent is detected. The sensor may deactivate a fan when agent concentration falls below a minimum amount.

The device may formed as a mobile trolley.

In some embodiment the device may be wall mountable.

A further aspect provides a mobile anaesthetic gas scavenging device comprising a device as described herein.

A further aspect provides an anaesthetic gas capture system for extracting anaesthetic gas from the exhaust of an anaesthesia machine, the system comprises an extraction device, the device comprises or can be connected to suction means for drawing gas exhausted from a machine to the device to cause gas to flow through a capture canister, the system comprises an open reservoir from which the suction means draws gas, the machine exhausts into the open reservoir, whereby the device is pneumatically decoupled from the machine so that the device cannot apply vacuum pressure to the machine, the suction means is provided upstream of the capture canister.

The device may be connected or connectable to a gas scavenging system.

The connection from the device to the scavenging system may include a pneumatic break.

A further aspect provides an anaesthetic gas capture system, the system comprises an anaesthetic agent extraction device, the device comprises or can be connected to suction means for drawing gas including anaesthetic agents thereinto for extraction, the device is pneumatically decoupled from the source of the gas, the suction means is configured so that agent extraction occurs under positive pressure.

The device may draw gas from an anaesthetic machine.

The device may draw from a patient

The device may draw from a patient mask.

The device may draw from a medical environment (such as an operating theatre in a hospital or veterinary surgery).

A further aspect provides an anaesthetic gas capture device for extracting anaesthetic gas from an anaesthesia machine, the device comprises or is connectable to a suction means for causing gas flow from the machine through the device under positive pressure, the device comprises or can be connected to an air break for pneumatically decoupling the device from the machine so that a generally constant volumetric gas flow is maintained regardless of pneumatic variation caused by the machine or the device.

A further aspect provides an anaesthetic gas capture device for extracting anaesthetic gas from the exhaust of an anaesthesia machine, the device is connected or connectable to an anaesthesia machine, the device comprises or is connectable to a suction means for causing gas flow from the machine through the device, the device comprises or can be connected to an air break for pneumatically decoupling the device from the machine so that a generally constant volumetric gas flow is maintained regardless of pneumatic variation caused by the machine or the device.

In some embodiments a fan turns on when anaesthetic agent is detected upstream thereof.

An AGSS may exert negative pressure on the system.

The AGSS may try to pull more flow than is required/wanted for a device. Under these circumstances the AGSS can still pull what it needs.

An air break line may be provided in some embodiments.

An air break may have access to environmental air. Under certain conditions it may draw in atmospheric air.

An air brake may close when a fan, for example, stops and it may be open when the fan is running.

When the air brake is closed the AGSS can pull unimpeded.

An air brake may be used to limit air flow into the capture system. For example an air brake may be useful to prevent an AGSS from pulling more air flow through capture container/s than they can handle.

A fan (not the AGSS) may, for example be used to drive gas flow through capture containers.

A ball type flow indicator may be provided to show if the AGSS is pulling.

Devices formed in accordance with the present invention have no effect on a patient.

An aspect of the present invention provides an anaesthetic gas capture rig for extracting anaesthetic gas from an anaesthesia machine, the rig comprises or can be connected to suction means for drawing gas exhausted from a machine to the rig, the rig is pneumatically decoupled from the machine so that the rig cannot apply vacuum pressure to the machine.

An aspect of the present invention provides an anaesthetic gas capture rig for extracting anaesthetic gas from the exhaust of an anaesthesia machine, the rig is connected or connectable to an anaesthesia machine, the rig comprises or is connectable to a suction means for causing gas flow from the machine through the rig, the rig comprises or can be connected to an air break for pneumatically decoupling the rig from the machine so that a generally constant volumetric gas flow is maintained regardless of pneumatic variation caused by the machine or the rig.

The rig may also be connected or connectable to a gas scavenging system, for example an Anaesthetic Gas Scavenging System (AGSS) in a hospital.

The rig may be pneumatically decoupled from the scavenging system.

In some scenarios, therefore, the rig sits between an anaesthetic machine and a scavenging system; it is decoupled on either side so its presence has no pneumatic effect on the overall system.

In some embodiments pneumatic decoupling at either or both sides of the device is achieved by a physical gap and/or by conduits/pipes/hoses with vent holes. For example, in some embodiments the air exhaust line from an anaesthetic machine terminates in a reservoir (e.g. a bucket) that is open to the atmosphere. Then a suction line from the device collects exhaust gases from the reservoir. Because the anaesthetic gases are typically heavier than air they remain at or sink to the bottom of the reservoir, from where they can be collected. This means that suction from the device can never apply vacuum pressure to the anaesthetic machine or to a patient. Similarly, if the device is connected to a gas scavenging system a pneumatic break is also provided, meaning that the gas scavenging system cannot apply vacuum pressure to the device; this, together with the anaesthetic machine side break, provides a double safety feature. In some embodiments, for example the device may include a line for pushing waste gas to the scavenging system (having passed through capture canister/s). The waste gas line from the device may, for example, feed into a scavenging suction line. An open reservoir may, for example, be provided by having holes/gaps in the waste line and/or suction line. A double air break arrangement is therefore provided.

An aspect provides an anaesthetic gas capture system, the system comprises an extraction device, the device comprises or can be connected to suction means for drawing gas including anaesthetic agents thereinto for extraction, the device is pneumatically decoupled from the source of the gas.

The device may, for example, draw gas from an anaesthetic machine.

The device may, for example, draw from a patient. For example the device may draw from a patient mask. A pneumatic break ensures that the device cannot apply suction directly to the patient.

The rig may comprises or has means for receiving one or more capture canisters through which exhaust gas flows, the canisters contain filter material for capturing anaesthetic agent from the gas flow. For example the rig may comprise one or more bays for receiving removable capture canisters. When a canister becomes full it can be removed.

In some embodiments canisters can be removed/installed during operation of the rig.

A valve system may be used to allow canisters to be removed during operation of the rig i.e. it is not necessary to turn the rig off to remove/install a canister.

The rig may comprise means for determining the initial and/or remaining capacity of a canister.

Some embodiments include the use of one or more capture canisters which receive waste gas. For example, waste gas may be diverted from the exhaust of the anaesthetic machine. The waste gas may flow into an ingress pipe that feeds into a capture canister.

The anaesthetic machine from which the canister receives waste gas may deliver several different types of agent. Accordingly, the canister may collect a mixture of volatile anaesthetic agents.

In some embodiments halocarbons are captured onto a filter material contained in a canister from the exhaust of the anaesthetic machine.

Filter materials may, for example, include Silica (SiO2), zeolites, carbon and modified or unmodified silica-based or cellulosic aerogels.

Some embodiments of the present invention relate to a capture rig for housing one or more capture canisters.

When the filter material in a canister is saturated with agent, the feed of waste gas to the ingress pipe may be terminated and the canister removed from an ingress pipe and/or an egress pipe.

Alternatively or additionally a "hotswap" system may be provided. Canister valves may, for example, be provided to enable hotswapping.

A device formed in accordance with the present invention may be a free-standing unit capable of containing up to four (for example) cylinders at a time.

The suction means may comprise one or more of a vacuum source, a venturi pump or a fan.

One or more fans may be provided, for example. The fan/s may be used to regulate flow rate, for example using a fan tachometer.

In some embodiments the suction means comprises a plurality of (for example two) fans. This could, for example, be used to build in redundancy to the system.

The present invention also provides a mobile anaesthetic gas scavenging device comprising a rig as described herein.

Materials may be selected so that the apparatus can cope not only with anesthesia gases but also high concentrations of O₂.

A lifting mechanism may be provided.

Some embodiments relate to a capture rig for housing a plurality of capture canisters.

Some embodiments may work with different types of anaesthetic machines e.g. open reservoir and integral weigh cell.

A bypass arrangement may be provided.

The canister/s may be able to withstand temperatures and/or pressures required for subsequent extraction of agent bound to the filter material.

The canister/s may be able to withstand temperatures and/or pressures required for subsequent supercritical fluid extraction of agent bound to the filter material.

Alternatively or additionally a pressure-intolerant sleeve containing filter material for capturing one or more types of anaesthetic halocarbon prior to extraction (e.g. supercritical fluid extraction) may be provided. A pressure-tolerant housing into which the sleeve can be inserted may also be provided so as to permit exposure of the sleeve contents to pressures required for extraction.

Some aspects embodiments relate to the capture of volatile anaesthetic agent and their separation and purification for the purposes of re-supply. This 'remanufacture' process is intended to provide financial and environmental cost savings.

Optional features:
- an open reservoir (size to be determined by risk of potential breakthrough)
- 1, 2, 3, 4 canisters
- Potential duplex fan
- position of inlet and outlet
- what the user interface could be • how to indicate 'no canister' for hotswapping

Some embodiments may discourage taking out all cylinders in IFU (instructions for use).

Emergency swapping may be addressed during training and reflected in instructions printed on the door of the Unit.

In some circumstances there may be a risk presented by switching on without any cylinders - but if it is necessary to continue in an emergency, operations should happen with full knowledge that venting through AGSS.

There may be an indicator for canister 'present / not present'; it may be preferable not to rely on the AGSS (as in some systems this may not be present).

### Open reservoir

Avoid/prevent potential of breakthrough of volatile at high O2 flow.

rate. 85l/mins max, fan draw at x litres, 20 seconds.

### Trolley 4 cylinders

The product may be 1-4 cylinders/canisters and mounted on a trolley.

An alternative embodiment may be wall mounted and could, for example, have 2 canisters.

In some embodiment a 4-canister model may be used for both wall and trolley mounted as this may increase the safety margin with respect to back pressure. In view of this, it may be possible to have essentially the same unit, either wall or trolley mounted.

### User Interface format - simple, could be LED or small screen

May be traffic lights.

Could be like a fuel tank, or five bars, or needle gauge, for example.

May be connected to weigh cell so measures weight.

Not interactive, only displays capacity.

May provide for fluorocarbon measuring.

Electronics may reset when canister is replaced.

Canister 'present / not present' indicator.

### Implement dual fan/duplex

If possible, if does cause other problems or risks Inlet/outlet position and AGSS pipe.

This is a usability question - may be best to design from this point of view e.g. both inlets may be clearly labelled together, with different connections.

AGSS pipework is heavy so near the base may be preferable.

### Regulatory requirements

The capture rig may comply with ISO 13485 Class 1a regulations.

Some embodiments allow fitment of 2 (for example) cannisters in parallel.

Capacity for 4 cylinders rather than 2 may be provided.

Inlet/outlet ports may be located at the bottom of the product.

Duplex fans may be provided.

A user interface may be provided.

The apparatus may be provided as a floor standing product.

Regarding the gas flow, the open reservoir may be removed but with scope to fit the part using the same mouldings. In some embodiments the proposal is that this port can be 'capped' on early release products, if there is a future requirement for an open reservoir to work in conjunction with anesthesia machines operating in a passive setup (anesthesia machine has no open reservoir) this part could be retrofitted with little impact. This update may be implemented. In >90% of cases the anesthesia machine will be fitted with an open reservoir. Two open reservoirs in series would not allow for extraction of gas.

### Option 1 - Open reservoir not fitted

In this case the open reservoir fitted to the anesthesia machine will assume the role of our reservoir, so it is not required. NRV1 is the bypass positioned directly between the inlet and exhaust ports to the product, the bypass must be positioned before the fans on the outlet. Fans 1 and 2 shown to deal with the requirement for redundancy. NRV2 is effectively and air admittance valve which serves the AGSS, gas will be driven through the system by the fan into the AGSS air stream if present. If AGSS is not present, gas will be driven to the exhaust solely by the fans. There is no requirement for the user to make any changes to the system in either scenario. PRV1 is present to vent in case of positive pressure, this could be caused by failure to fit an exhaust pipe or a buckled exhaust pipe. CM H2O values are indicative.

### Option 2 - Open reservoir fitted

Option 2 illustrates the fitment of the open reservoir for use with 'passive' setups. As mentioned previously, the open reservoir in this case will deal with O2 flush and fan draw to ensure no impact on the anesthesia machine. The rest of the system remains the same and so supports retrofitting of an open reservoir 'cartridge'.

The schematic layouts on the following pages detail how the system could cope with various scenarios. These could be a result of cannister removal, blockage or incorrect use. The system should always fail safe. These scenarios have been illustrated with no open reservoir fitted to the device; green lines represent anesthesia gas flow.

### Scenario 1 - Normal operation with AGSS

AGSS draws through NRV 2, Fan draws gases through the cannisters and into the AGSS air stream.

Scenario 1 - Normal operation with AGSS (fans in push configuration) with silencer.

The fan is positioned at the top of the canister, "pushing" the gas rather than "pulling"). A silencer is included.

### Scenario 2 - Normal operation without AGSS.

Fans draw gases through the cannisters and out to exhaust.

### Scenario 3 - Bypass

Gases bypass cannisters and run directly to exhaust from the input, this scenario could occur because of cannister removal, blockage or failure to fit exhaust pipe.

### Scenario 4 - Exhaust blockage

Gases exit through PRV1 (to local environment) in the event of blockage on exhaust. Causes - failure to fit exhaust pipe, buckled pipe.

Some embodiments make use of load cells and capacitive sensors to check the capacity,

Some embodiments have the ability to 'split' the manifold and effectively float each cannister to take individual readings.

In some embodiments the idea is that the capacitive sensor can be used to verify the removal or replacement of a cannister, it would require a reading from both the capacitive sensor and load cell to confirm canisters have been loaded correctly. Looking deeper into the logic of this system, it is also possible that we could track the capacity of individual cannisters. This is explained in more detail below.

In this scenario, the system starts with no cannisters fitted. The user comes along and fits four (for example) cannisters. Each of these cannisters will have a tolerance on their mass, for the purpose of this illustration we have assumed a fairly large tolerance of 2.00%, we will use this tolerance on the full capacity lower limit and empty capacity upper and lower limit.

The first check the system must perform is to confirm the cannister sits within the empty capacity tolerance. If it does, it can set the current capacity counter to 0 and confirm that the cannister has been correctly loaded on the UI. If not, there is the option to reject the cannister or determine the remaining capacity by deducting the empty capacity upper limit value from the measured mass.

The next step is to start measuring, the load cells are now tracking the change in mass and counting up on the current value.

Once the capacity reaches the set limit, the UI can indicate the cannisters require changing. This is all fairly straight forward on a system running with all four cannister filling at the same rate.

With the four capacitive sensors and load cells working together there may also be the ability to track the capacity of individual cells. This could be particularly useful in a scenario where the user has decided to change only one cannister (this isn't recommended behaviour as this will not encourage the gas to bias empty cannisters, but it does illustrate the versatility of the system).

In this scenario, the user has started running the system with a set of four cannister that are almost empty. They have continued to use the system and a previous reading has been recorded.

The capacitive sensor reads 0 to indicate that cannister two has been removed. At this point the system knows that the value of load cell has reduced but it also knows that a cannister has been removed. The previous reading of the cannister that has been removed could then be deducted from the previous total so it has no bearing on the running total of the other cannisters.

Cannister two may, for example, be replaced. The initial checks will be carried out and the current reading forced to 0. The system will then continue as normal dividing the total load cell reading across all cannisters relative to their previous reading if they're not been removed from the system.

Some embodiments build on other embodiments, with the addition of two more cylinders, the digital UI and reconfiguration of the inlet and outlet ports.

The digital UI is designed as monitor only, one change the user may wish to make is to switch between light and dark themes. This seems to be typical of monitors placed in theatre particularly for key hole surgery where excess light might be undesirable. An alternative might be to lead with a single setting rather than allowing the user to switch but this can be determined as the product develops.

Access to cannisters is achieved by opening the front panel. The safety valves and cavity for an open reservoir will also be revealed at this point so that users can perform checks ahead of starting a procedure.

Each cannister could be numbered for easy identification of specific cannister if they need to be changed individually. The valve at the base of the cannister should be designed to support the load of the cannister at full capacity plus compressive force of 'x' for good seating on the seal. The longer the free length of the spring the lighter the spring we can use to achieve 40mm travel for cannister removal (Travel distance will have a direct impact on the total height of the device).

The inlet and outlet ports have been moved to the base of the product to cope with the weight of the AGSS pipework. These could use standard BS and DISS fittings, these should be quite familiar to users of the anesthesia machines.

An alternative embodiment may be provided with a very different take on the construction of the product and user experience. The idea is that the cannisters could be accessed by a carrier lifting out of the base of the product, this lifts the cannisters to a comfortable working height. The manifold would split as the linear actuator raises the cannisters (could also be achieved with gas springs or other mechanisms but requires more consideration).

This embodiment allows for a much smaller form factor; this is down to routing of internal pipework and presenting the safety valves on the outside of the product rather than concealing them within. Functionality remains the same as it relies on the same schematic but the physical arrangement differs.

Inlet and outlet ports may be positioned low on the product but both the gas ports and safety valves are located on the side rather than front face of the product. This may help with pipe routing within a theatre.

Some embodiments display instructions on an inside panel of the door. The same approach could be applied to flat faces of the carrier if the cannisters are lifted.

Further aspects and embodiments are provided in the following numbered paragraphs.
1. An anaesthetic gas capture device for extracting anaesthetic gas from an anaesthesia machine, the device comprises or can be connected to suction means for drawing gas exhausted from a machine to the device, the device comprises one or more capture canisters through which exhausted gas can flow, the device is pneumatically decoupled from the machine so that the device cannot apply vacuum pressure to the machine, the device comprises a sensor for sensing the presence of anaesthetic agent in gas flow from the machine, the sensor is provided on an inlet side of the device, the suction means is provided upstream of the capture canister/s and downstream of the sensor, in which the sensor can activate the suction means when anaesthetic agent is detected, whereupon the suction means pushes gas through the or one or more of the canisters under positive pressure.
2. The device of paragraph 1, in which the device is also connected or connectable to a gas scavenging system.
3. The device of paragraph 1 or paragraph 2, in which the device is also connected or connectable to an Anaesthetic Gas Scavenging System (AGSS).
4. The device of paragraph 2 or paragraph 3, in which the device is pneumatically decoupled from the scavenging system.
5. The device of any preceding paragraph, in which the device comprises or has means for receiving one or more capture canisters through which exhaust gas flows, the canisters contain filter material for capturing anaesthetic agent from the gas flow.
6. The device of paragraph 5, in which the or each canister can be removed/replaced during operation of the device.
7. The device of paragraph 5 or paragraph 6, comprising means for determining the remaining capacity of a canister.
8. The device of any of paragraphs 5 to 7, comprising means for detecting the presence of a canister.
9. The device of any preceding paragraph, in which the suction means is one or more of a vacuum source, a venturi pump or a fan.
10. The device of any preceding paragraph, in which the suction means comprises a plurality of fans.
11. The device of any preceding paragraph, comprising an air break formed within or between flow conduits.
12. The device of any preceding paragraph, comprising a bypass to receive flow when the suction means in inactive and/or to act as a high pressure bypass.
13. The device of any preceding paragraph, in which the sensor activates and/or deactivates the suction means based on detection of a threshold agent concentration or amount.
14. The device of any preceding paragraph and formed as a mobile trolley.
15. The device of any preceding paragraph and being wall mountable.
16. A mobile anaesthetic gas scavenging device comprising a device as claimed in any preceding paragraph.
17. An anaesthetic gas capture system for extracting anaesthetic gas from the exhaust of an anaesthesia machine, the system comprises an extraction device, the device comprises or can be connected to suction means for drawing gas exhausted from a machine to the device to cause gas to flow through a capture canister, the system comprises an open reservoir from which the suction means draws gas, the machine exhausts into the open reservoir, whereby the device is pneumatically decoupled from the machine so that the device cannot apply vacuum pressure to the machine, the suction means is provided upstream of the capture canister.
18. The system of paragraph 17, in which the device is connected to a gas scavenging system.
19. The system of paragraph 18, in which the connection from the device to the scavenging system includes a pneumatic break.
20. An anaesthetic gas capture system, the system comprises an anaesthetic agent extraction device, the device comprises or can be connected to suction means for drawing gas including anaesthetic agents thereinto for extraction, the device is pneumatically decoupled from the source of the gas, the suction means is configured so that agent extraction occurs under positive pressure.
21. The system of paragraph 20, in which the device draws gas from an anaesthetic machine.
22. The system of paragraph 21, in which the device draws from a patient
23. The system of paragraph 22, in which the device draws from a patient mask.
24. An anaesthetic gas capture device for extracting anaesthetic gas from an anaesthesia machine, the device comprises or is connectable to a suction means for causing gas flow from the machine through the device under positive pressure, the device comprises or can be connected to an air break for pneumatically decoupling the device from the machine so that a generally constant volumetric gas flow is maintained regardless of pneumatic variation caused by the machine or the device.
25. An anaesthetic gas capture device for extracting anaesthetic gas from the exhaust of an anaesthesia machine, the device is connected or connectable to an anaesthesia machine, the device comprises or is connectable to a suction means for causing gas flow from the machine through the device, the device comprises or can be connected to an air break for pneumatically decoupling the device from the machine so that a generally constant volumetric gas flow is maintained regardless of pneumatic variation caused by the machine or the device.
26. An anaesthetic gas capture device for extracting anaesthetic gas from an anaesthesia machine, comprising suction means for drawing gas exhausted from an anaesthetic machine to the device, the device has means for receiving one or more capture canisters through which exhausted gas can flow, the device comprises an air break such that it cannot apply vacuum pressure to the machine in use, the device comprises a sensor for sensing the presence of anaesthetic agent in gas flow from the machine, the sensor is provided on an inlet side of the device, wherein the suction means is provided upstream of the capture canister/s and downstream of the sensor, in which the sensor can activate the suction means when anaesthetic agent is detected, whereupon the suction means pushes gas through the or one or more of the canisters under positive pressure.
27. A capture canister for an anaesthetic gas capture machine, the canister comprises a body for receiving filter material and through which gas containing anaesthetic agent can flow, the canister includes an inlet and an outlet, a valve is provided for the inlet and the outlet, the inlet and outlet valves being configured to be normally closed to seal the body and configured to open when the canister is loaded into an anaesthetic gas capture machine.
28. A canister according to paragraph 27, in which the inlet valve and/or outlet valve include a return spring.
29. A canister according to paragraph 27 or paragraph 28, in which the inlet valve and/or outlet valve is formed as a poppet valve.
30. A canister according to any of paragraphs 27 to 29, comprises a mesh plate at or towards the inlet and/or outlet.
31. A canister according to paragraph 30, in which part of the valve is positioned in the mesh.
32. A canister according to paragraph 30 or paragraph 31, in which the valve is supported by the mesh.
33. An anaesthetic capture device for extracting anaesthetic agent from an anaesthetic machine, the device can releasably receive one or more capture canisters through which gas containing agent can flow.
34. A device according to paragraph 7, comprising one or more canister loading mounts for releasably receiving capture canisters.
35. A device according to paragraph 33 or paragraph 34, in which the device is configured to push exhausted gas through the or one or more of the canisters under positive pressure.
36. A device according to any of paragraphs 33 to 35, in which the device comprises or can be connected to suction means for drawing gas exhausted from a machine to the device,
38. A device according to any of paragraphs 33 to 36, in which the device is pneumatically decoupled from the anaesthetic machine so that the device cannot apply vacuum pressure to the machine,
39. A device according to any of paragraphs 33 to 37, in which the device comprises a sensor for sensing the presence of anaesthetic agent in gas flow from the machine,
40. A device according to paragraph 39, in which the sensor is provided on an inlet side of the device,
41. A device according to any of paragraphs 36 to 40, in which the suction means is provided upstream of the capture canister/s and downstream of the sensor.
42. A device according to any of paragraphs 39 to 41, in which the sensor can activate the suction means when anaesthetic agent is detected, whereupon the suction means pushes gas through the or one or more of the canisters under positive pressure.
43. A device according to any of paragraphs 33 to 42 configured to receive one or more capture canisters as claimed in any of claims 27 to 32.
44. A device according to any of paragraphs 33 to 43, in which the device is also connected or connectable to a gas scavenging system.
45. A device according to paragraph 44, in which the device is pneumatically decoupled from the scavenging system.
46. A device according to any of paragraphs 33 to 45, in which the or each canister can be removed/replaced during operation of the device.
47. A device according to any of paragraphs 33 to 46, comprising means for determining the remaining capacity of a canister.
48. A device according to any of paragraphs 33 to 47, comprising means for detecting the presence of a canister.
49. A device according to any of paragraphs 33 to 48 and formed as a mobile trolley.
50. A device according to any of paragraphs 33 to 49 and being wall mountable.
51. A mobile anaesthetic gas scavenging device comprising a device according to any of paragraphs 33 to 50.
52. A medical environment provided with one or more devices according to any of paragraphs 33 to 51.

Following capture the anaesthetic agent may be recovered from a capture canister, purified and re-used.

A medical environment, such as a hospital or veterinary facility, could be provided with one or more devices or systems as described herein.

Different aspects and embodiments of the invention may be used separately or together.

The present invention is also described, by way of example, with reference to the accompanying drawings.

All orientational terms, such as upper, lower, radially and axially, are used in relation to the drawings and should not be interpreted as limiting on the invention or its connection to a closure.

Example embodiments are described in sufficient detail to enable those of ordinary skill in the art to embody and implement the systems and processes herein described. **It** is important to understand that embodiments can be provided in many alternate forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed and as well as individual embodiments the invention is intended to cover combinations of those embodiments as well. On the contrary, all modifications, equivalents, and alternatives falling within the scope of the appended claims should be included. Elements of the example embodiments are consistently denoted by the same reference numerals throughout the drawings and detailed description where appropriate.

The terminology used herein to describe embodiments is not intended to limit the scope. The articles "a," "an," and "the" are singular in that they have a single referent; however, the use of the singular form in the present document should not preclude the presence of more than one referent. In other words, elements referred to in the singular can number one or more, unless the context clearly indicates otherwise. **It** will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof.

Unless otherwise defined, all terms (including technical and scientific terms) used herein are to be interpreted as is customary in the art. **It** will be further understood that terms in common usage should also be interpreted as is customary in the relevant art and not in an idealized or overly formal sense unless expressly so defined herein.

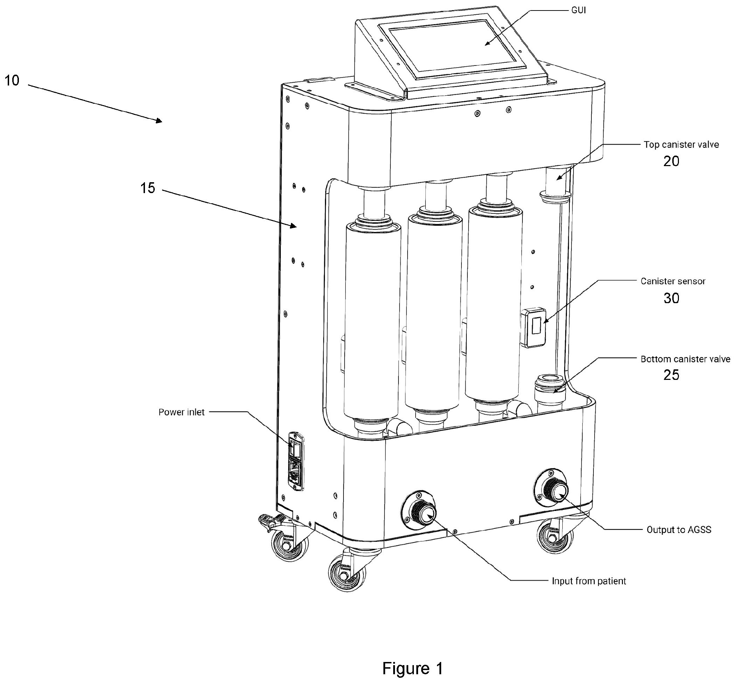

Figure 1 shows a capture rig 10 formed in accordance with an aspect of the present invention and comprising a main body 15. The body 15 has four (in this embodiment) pairs of top and bottom canister valves 20, 25. Canister sensors 30 are provided to detect the presence/absence of a canister in each canister bay. A graphical user interface (GUI) is provided on the body.

The anaesthetic gas capture rig 10 extracts anaesthetic gas from an anaesthesia machine. The rig comprises or can be connected to suction means for drawing gas exhausted from a machine to the rig. The rig is pneumatically decoupled from the machine so that the rig cannot apply vacuum pressure to the machine.

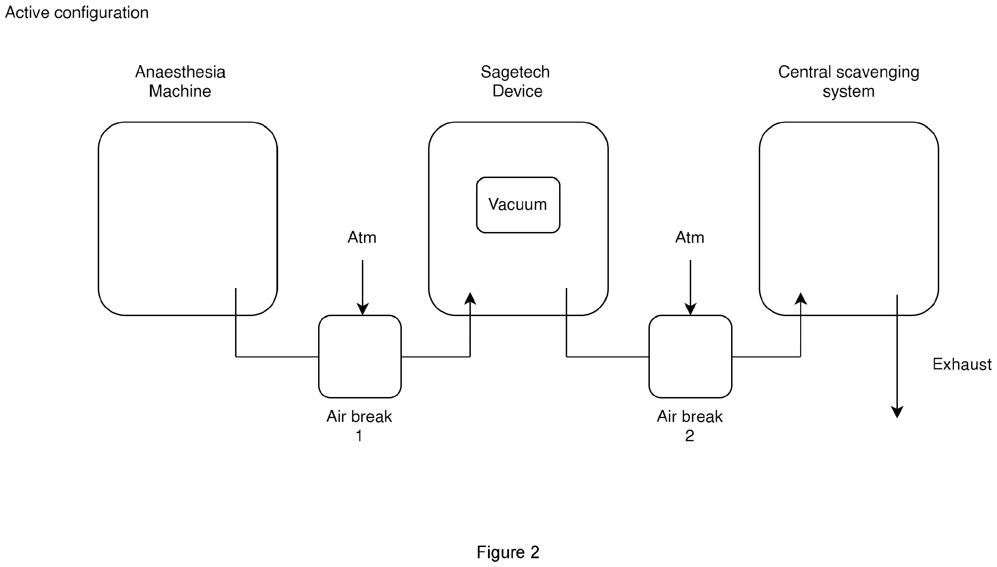

Figures 2, 3 and 4 show schematics for Active, Passive and Recovery configurations of aneasthesia systems including capture rigs ("Sagetech Device") formed in accordance with the present invention.

In the embodiment shown in Figure 2 an air break is in the device/rig (e.g. Air Break 2). In other embodiments an air break is integral to an anaesthesia machine.

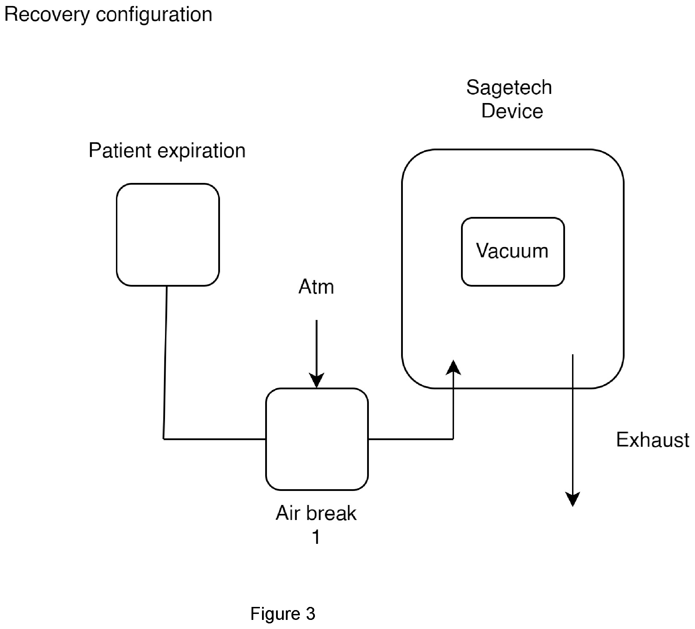

Figure 3 = Recovery Configuration.

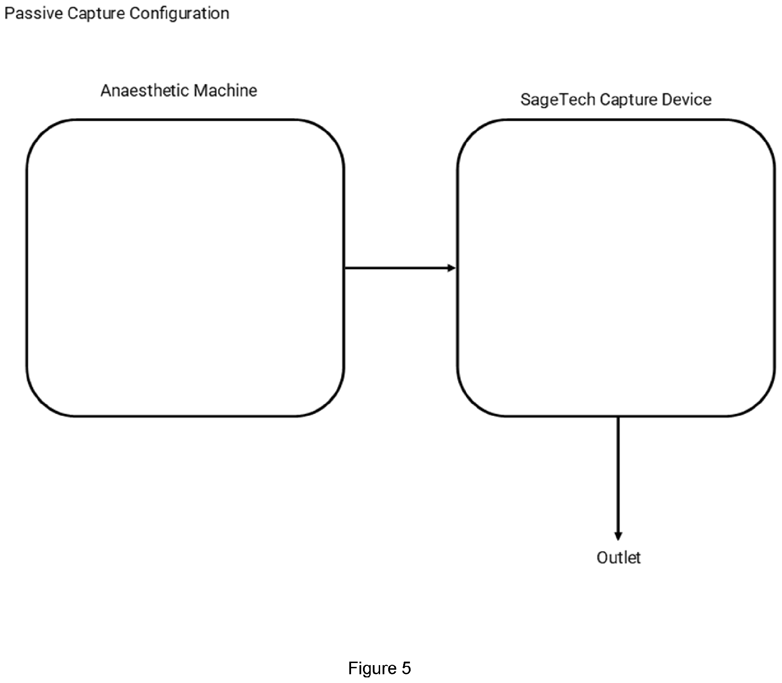

An alternative, Passive Configuration is shown in Figure 5.

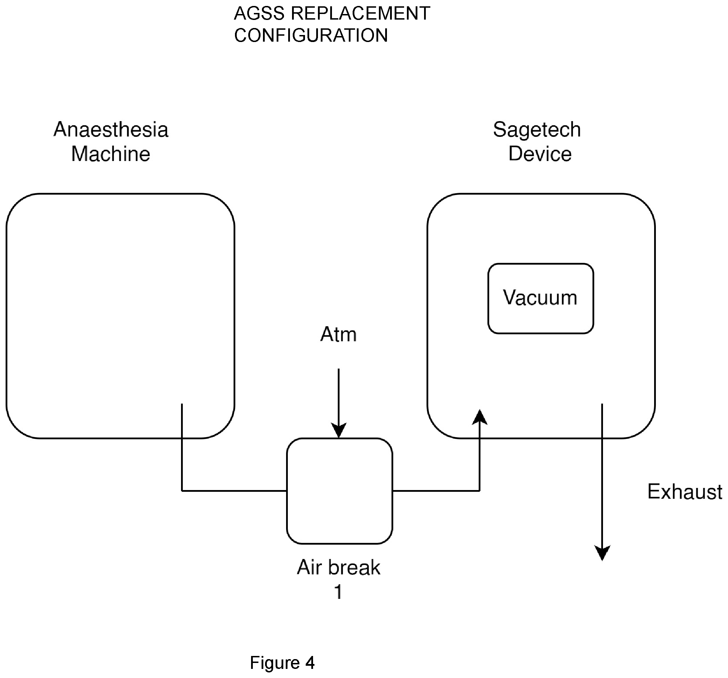

Figure 4 = AGSS Replacement Configuration.

In the schematics the rig has been shown with a vacuum source. This could be an external or internal vacuum source, venturi pump, or fans. The vacuum pressure may be between 10-20kPa vacuum pressure with a flow rate between 50-80lpm, for example. These values could increase or decrease if there are changes to legislation.

Either side of the device is an air break that effectively de-couples each of the systems so no over-pressure or vacuum condition can affect the patient.

The device is fitted with valves that allow canisters to be swapped while in operation. In a condition where all canisters have been removed, the system would move to a bypass condition so there is no interruption to gas flow through the systems.

Load cells and canister sensors work together to determine the presence and mass of individual canisters so that their capacity can be tracked and indication of when they need to be replaced can be provided.

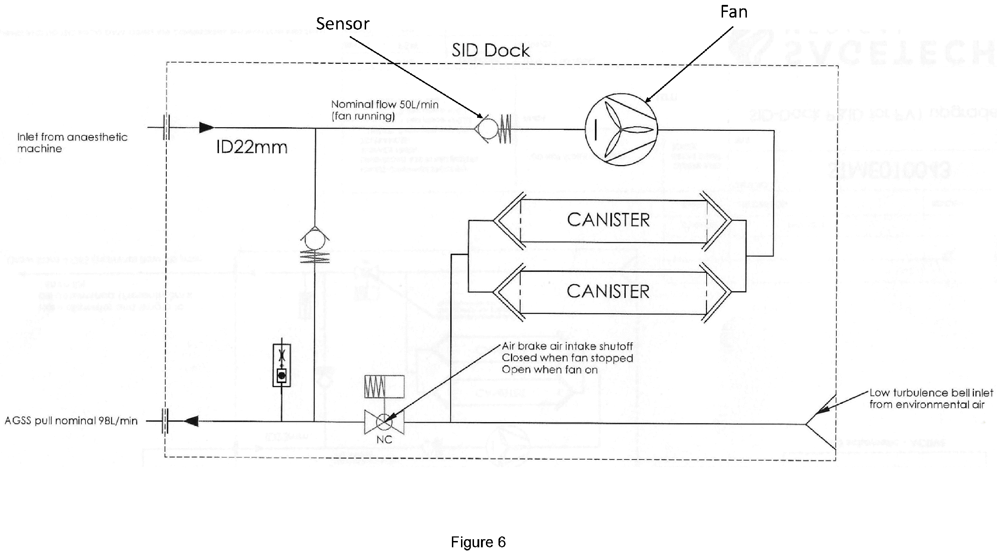

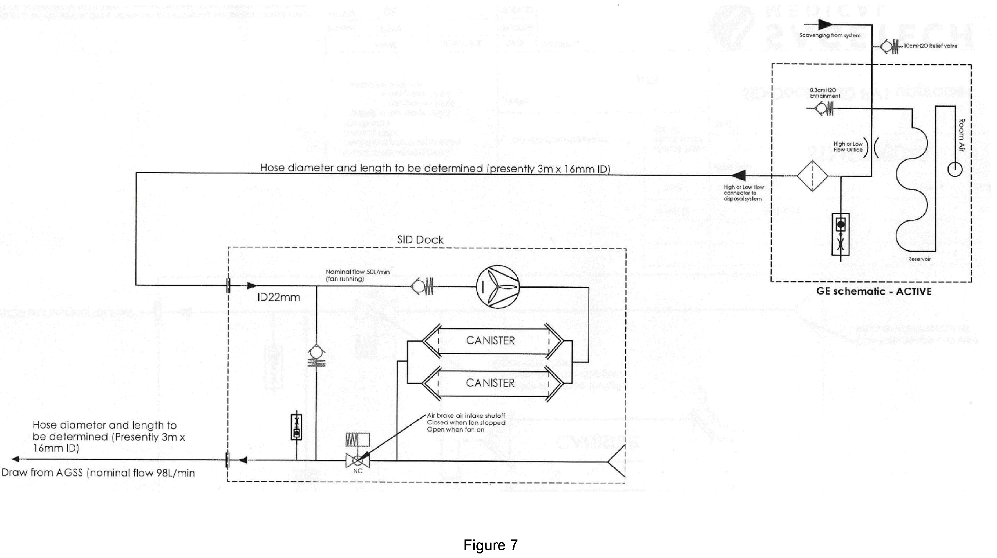

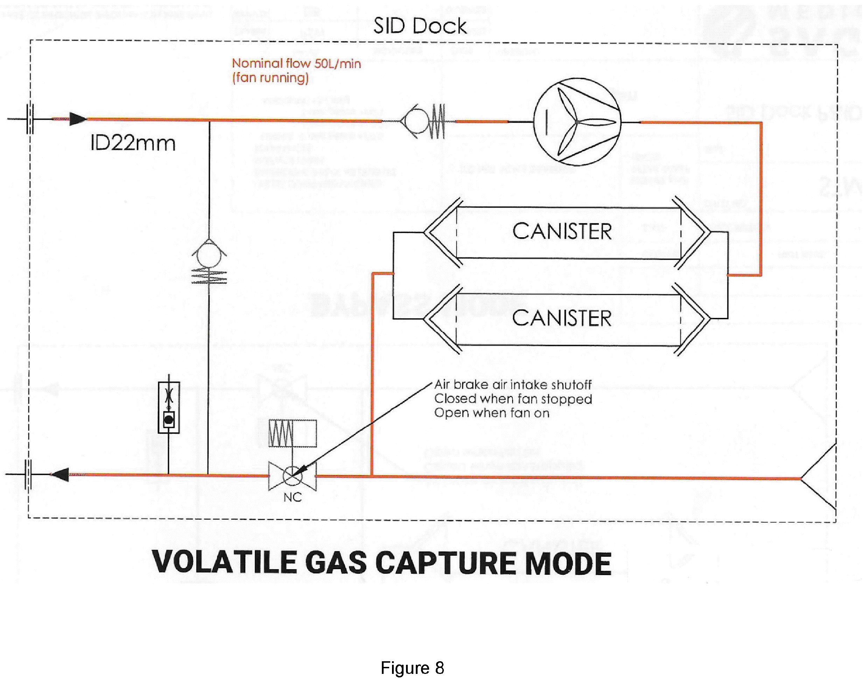

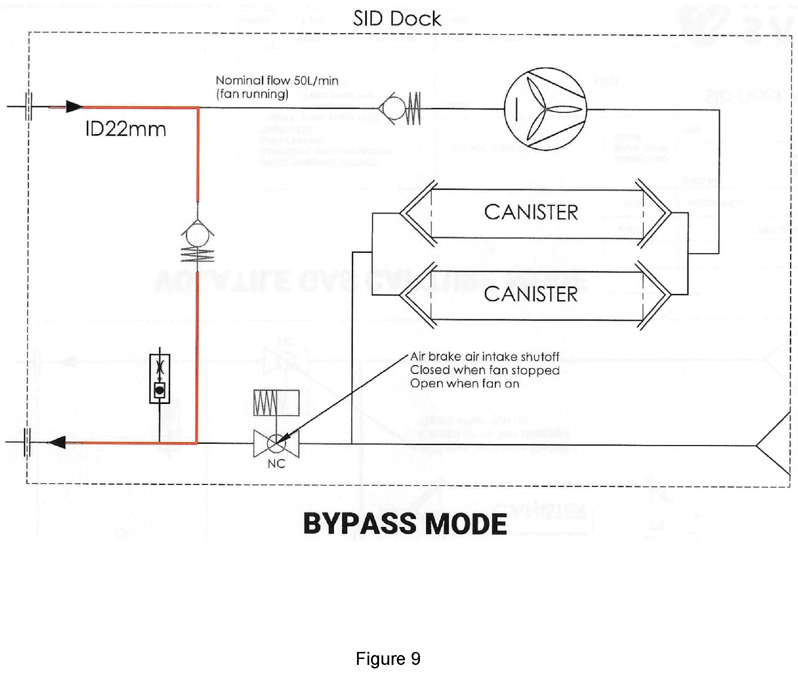

Figures 5 to 9 show schematic flow diagrams of a capture device formed in accordance with an embodiment the present invention.

An anaesthetic sensor (e.g. a VOC sensor) is provided at the inlet side of the device.

A vacuum source, in this embodiment a fan, is provided upstream of the canisters (and downstream of the sensor).

The sensor activates the fan when the level of VOC (anesthetic) detected in gas flow pulled from the anaesthetic machine is high.

Because the fan is at the inlet side of the canister it pulls flow from the anaesthetic machine and pushes gas through the canister.

This means the system is not pulling a vacuum across the canister. This results in more efficient binding of anaeasthetic agent onto the canister filter, because binding is happening in a positive and not a negative pressure environment. This also reduces duty of the vacuum source because it is not having to fight airway resistance provided by the filter in the canister.

In other embodiments, switching between different modes is achieved manually.

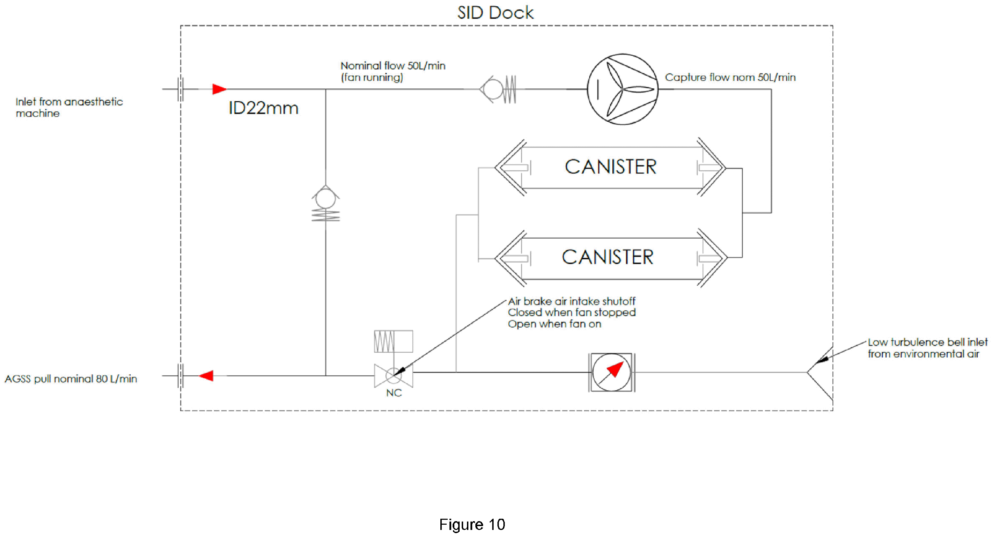

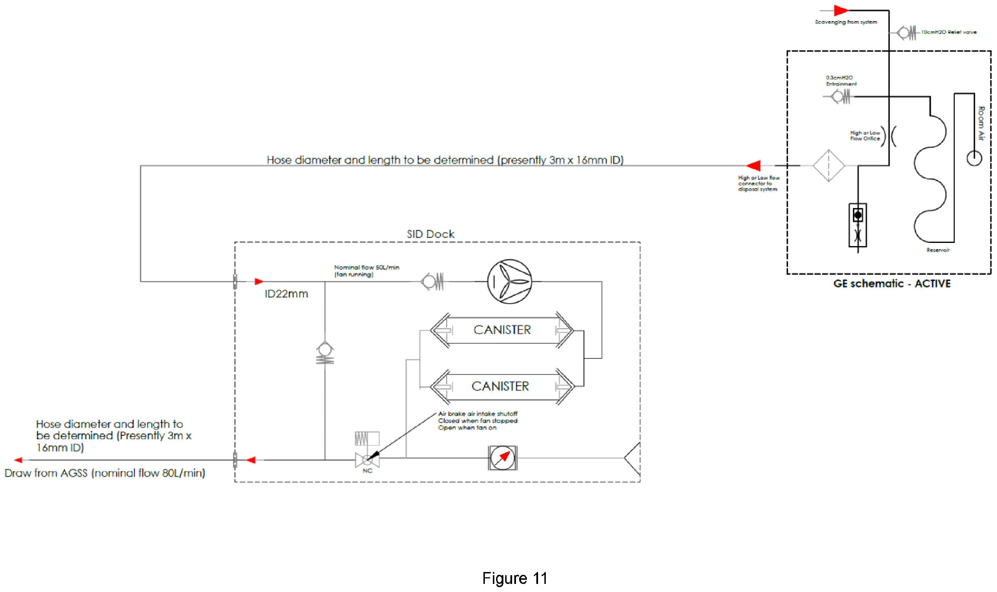

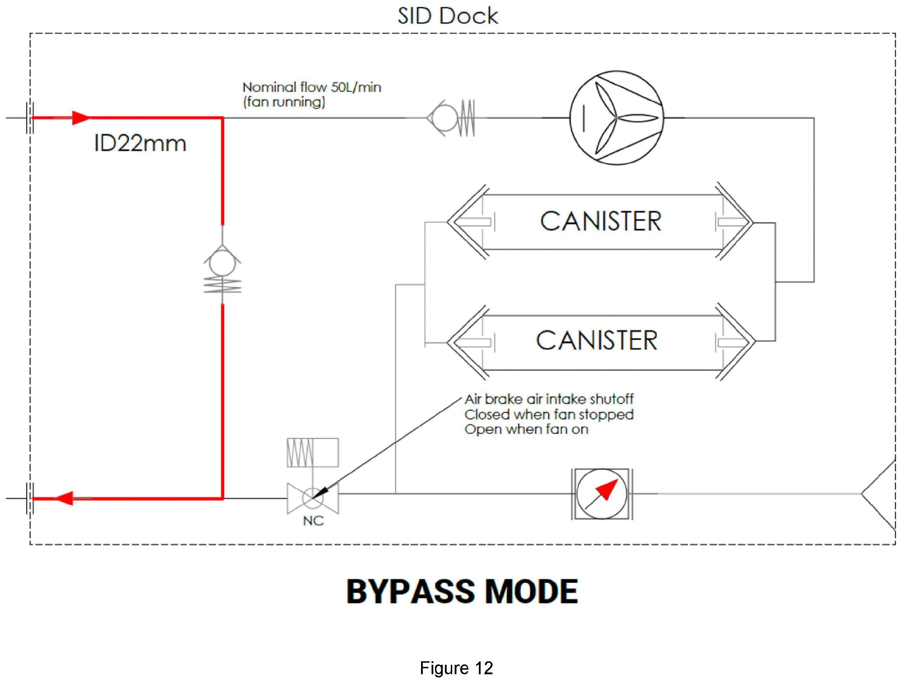

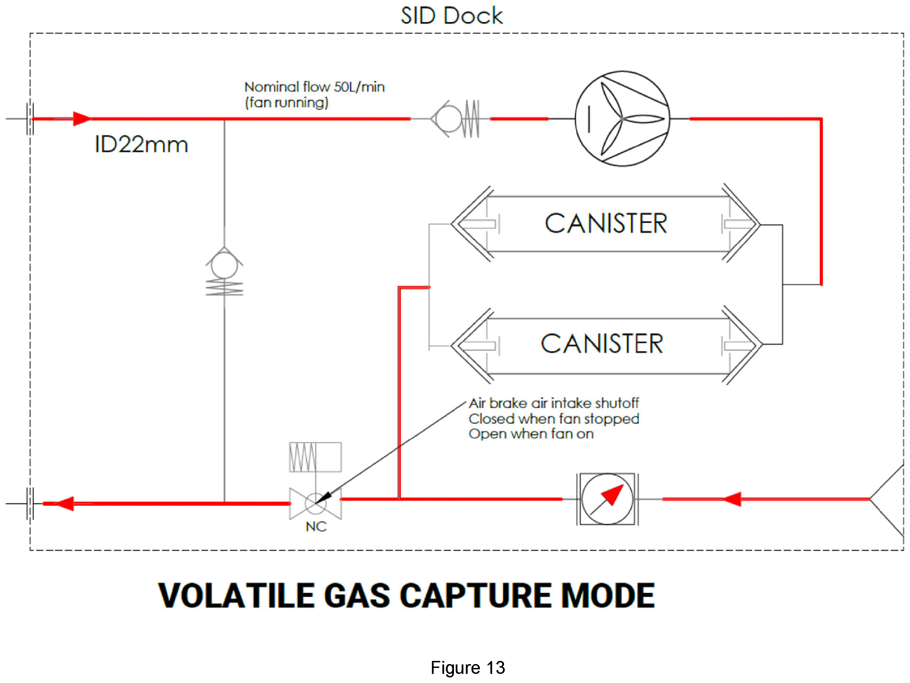

Figures 10 to 13 show schematic flow diagrams of a capture device formed in accordance with a further embodiment the present invention.

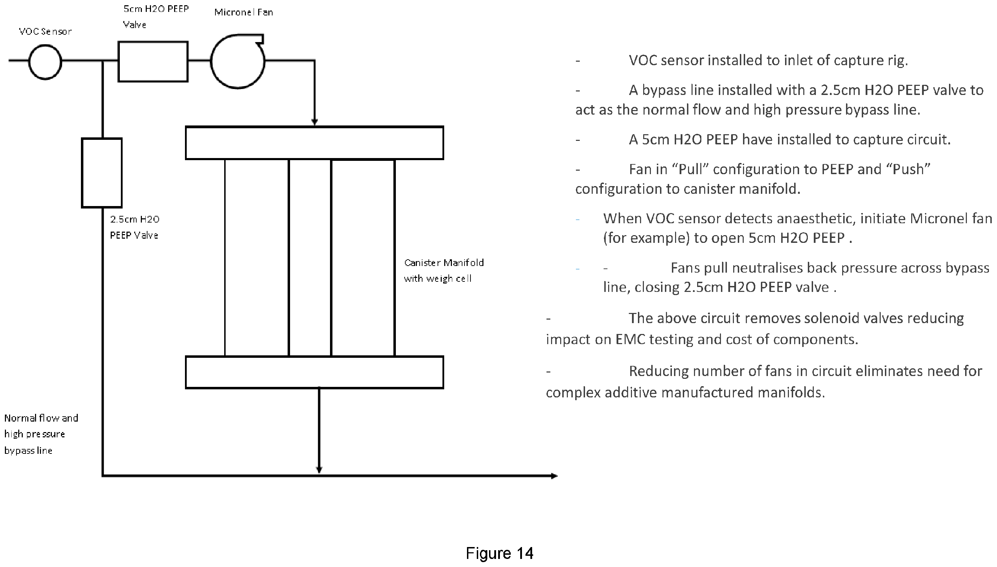

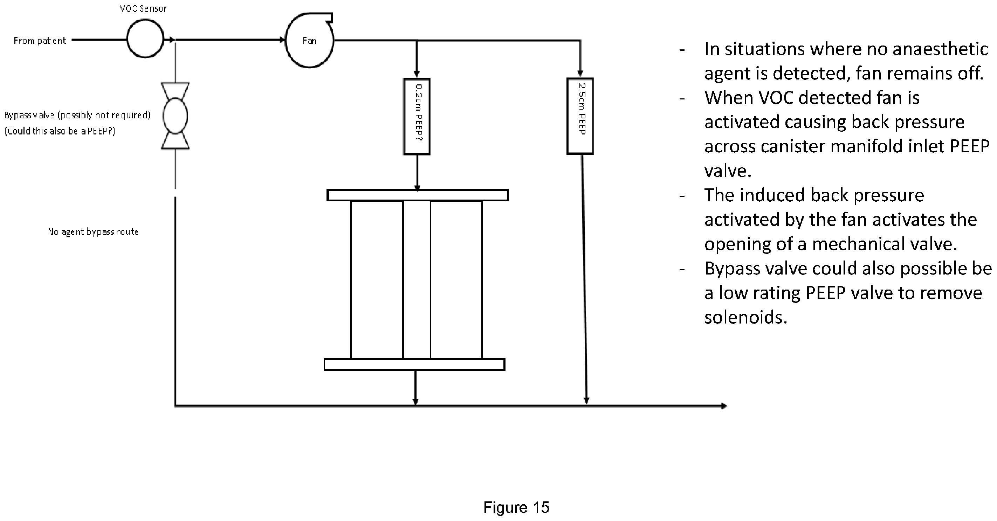

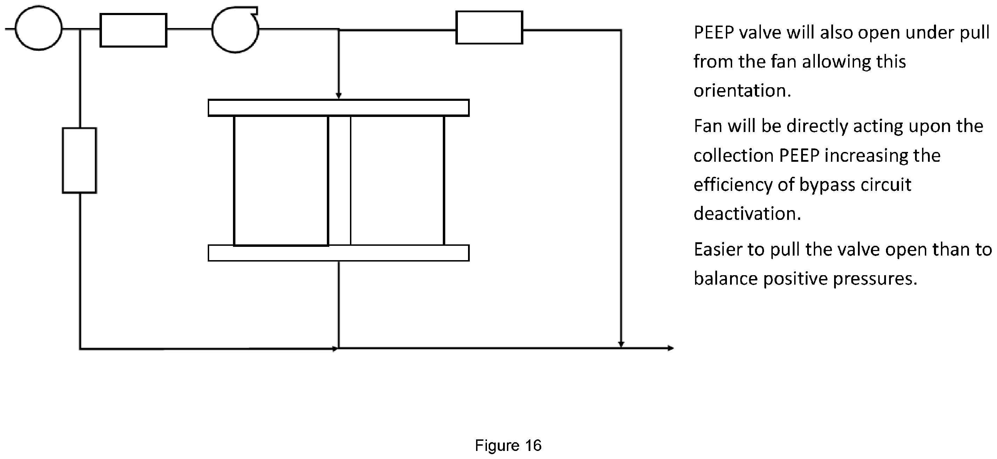

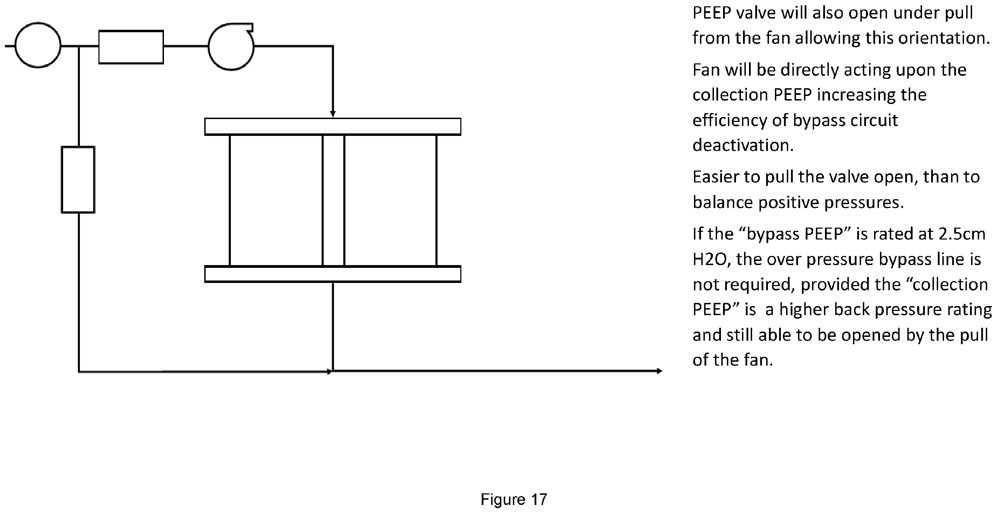

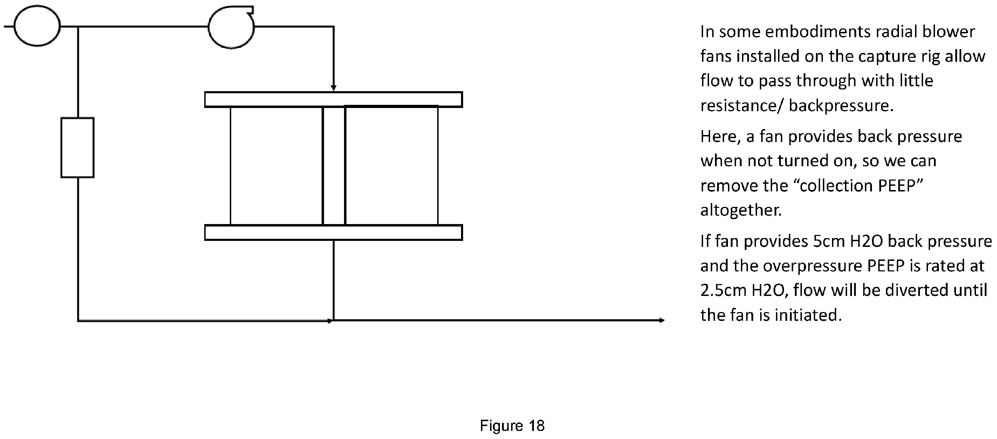

Figures 14 to 18 illustrate further embodiments.

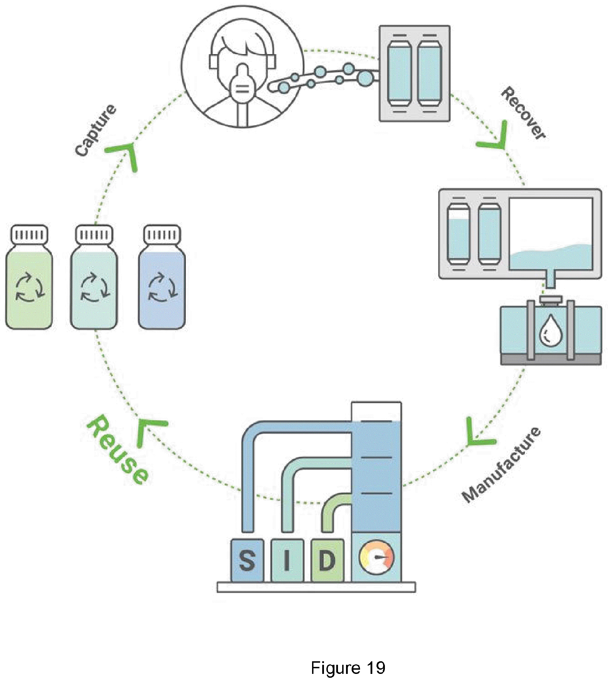

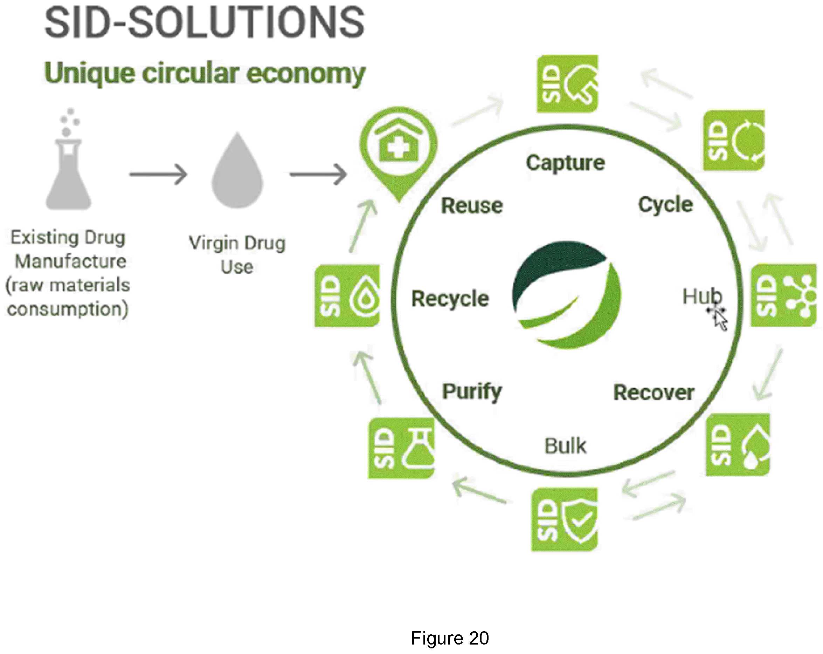

Figures 19 and 20 illustrate some of the principles underpinning some aspects and embodiments of the present invention.

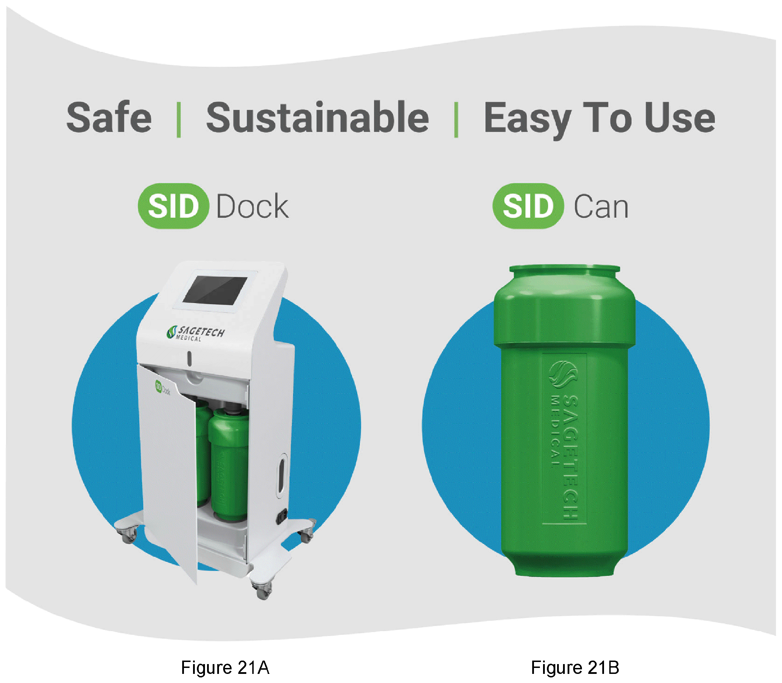

Figure 21A shows an extraction dock/machine/apparatus/appliance/device fitted with two capture canisters. Figure 21B shows a capture canister (removed from the dock).

Figure 22 shows the dock 100 with a front panel closed. Figure 23 shows the dock with the panel ajar, revealing a capture canister bay and fitted with two capture canisters 150.

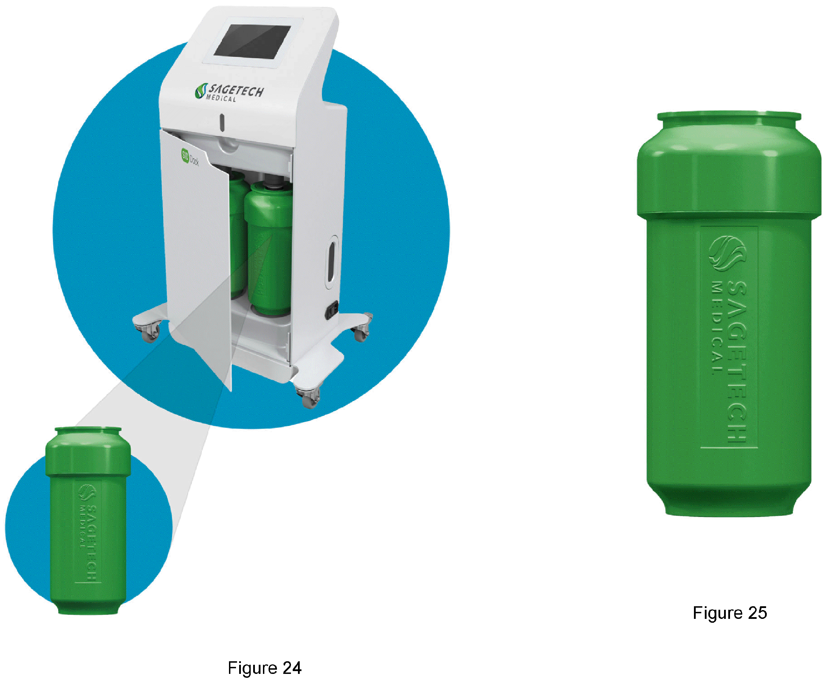

Figure 24 illustrates removal of a capture canister. Figure 25 shows a canister.

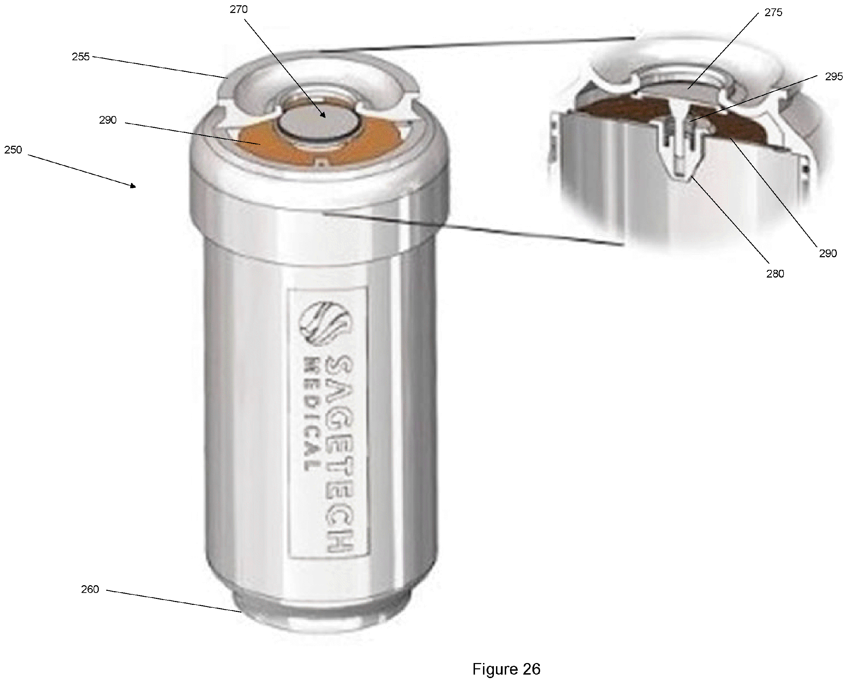

Figure 26 illustrates a capture canister 250 formed in accordance with an embodiment of the present invention.

### Safety

### SID-Can

Benefits:
- safe and easy to handle, store and transport
- intraoperative canister exchange ("hot-swap")
- lower number of changes

Features:
- light weight
- valves at both ends
- hydrophobic adsorbent material made from coconut husks captures 675ml (equals 2.7 bottles of agent)

Spring-loaded valves 270 are provided at both ends 255, 260.

The valve 270 comprises a plug part 275 and a socket part 280. The socket part 280 is held by a mesh plate 290. The mesh plate 290 also helps with flow distribution.

The plug 275 is resiliently biased to a closed position by a spring 295 and movable away from the closed position when loaded into a capture device. Spring-loaded activation of the canister valve is thereby provided.

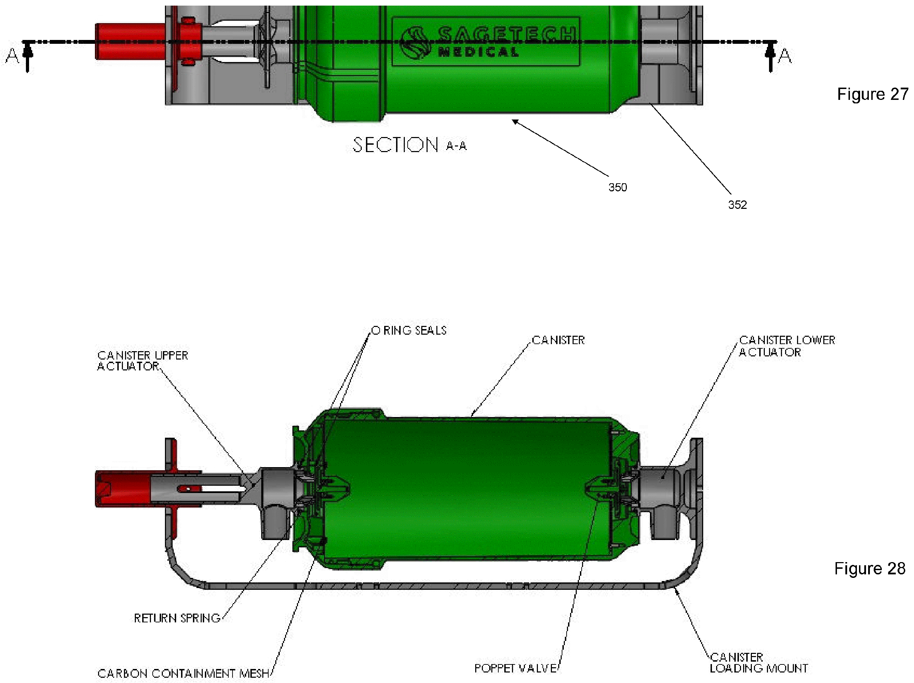

Figure 27 shows a canister 350 formed in accordance with the present invention and fitted into a canister loading mount 352 (that may be provided as part of a capture dock). Figure 28 is a section taken along line A-A of Figure 27.

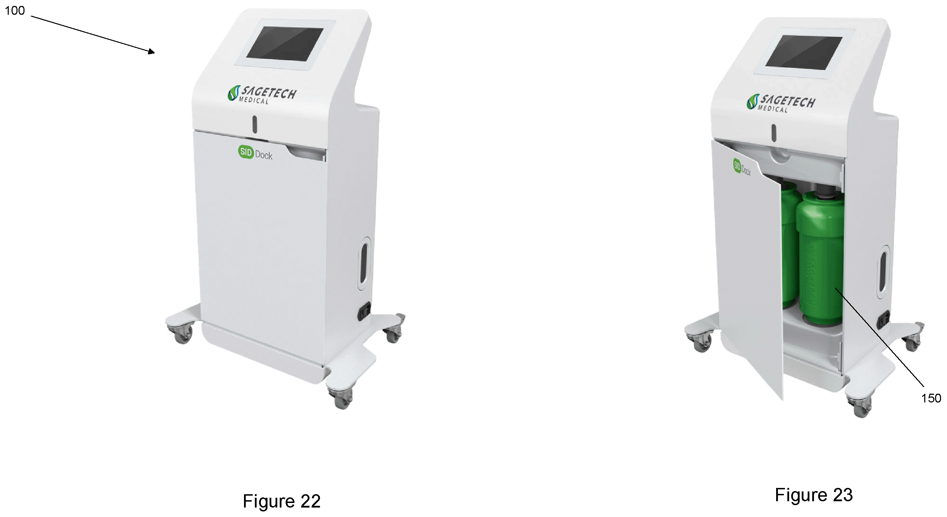

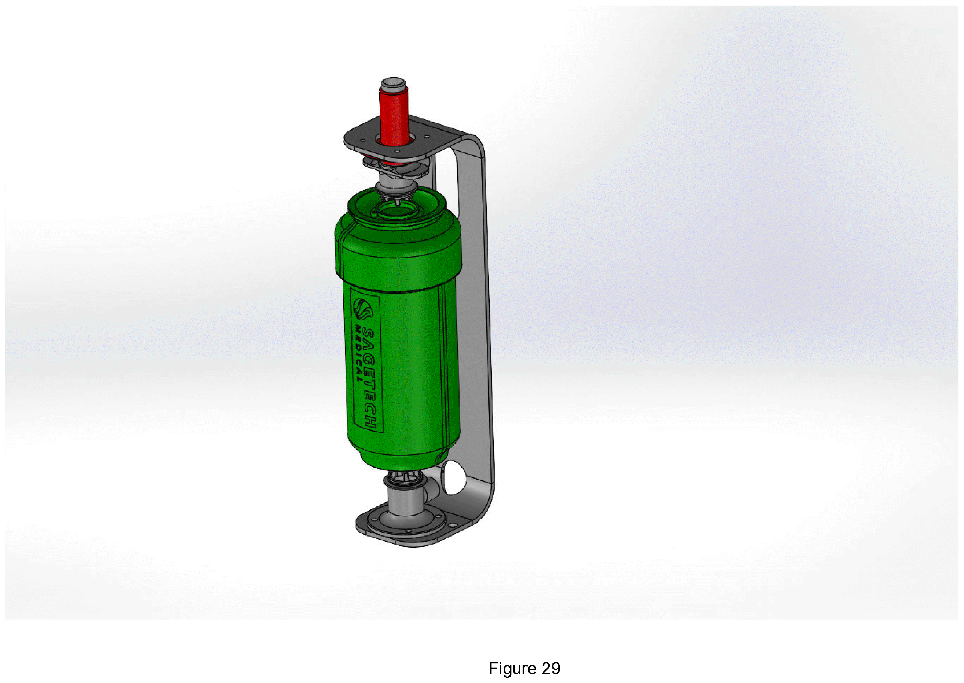

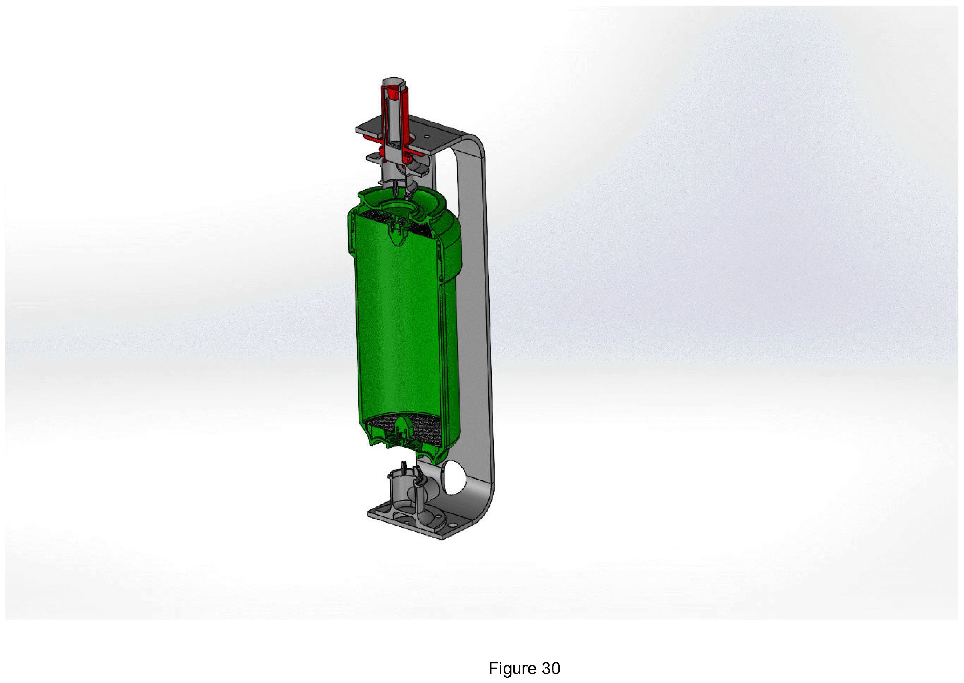

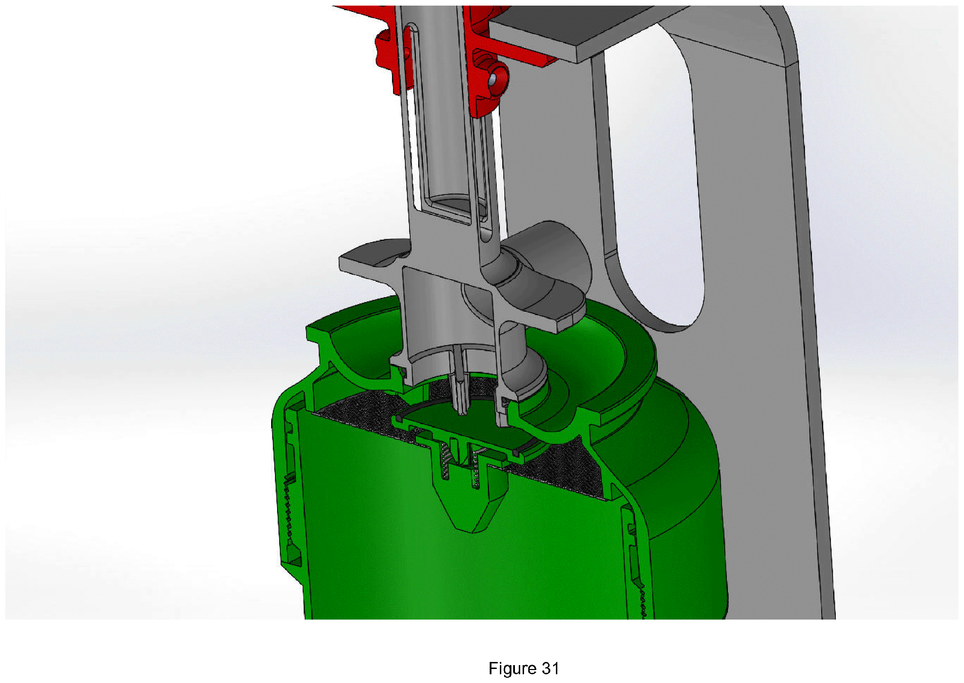

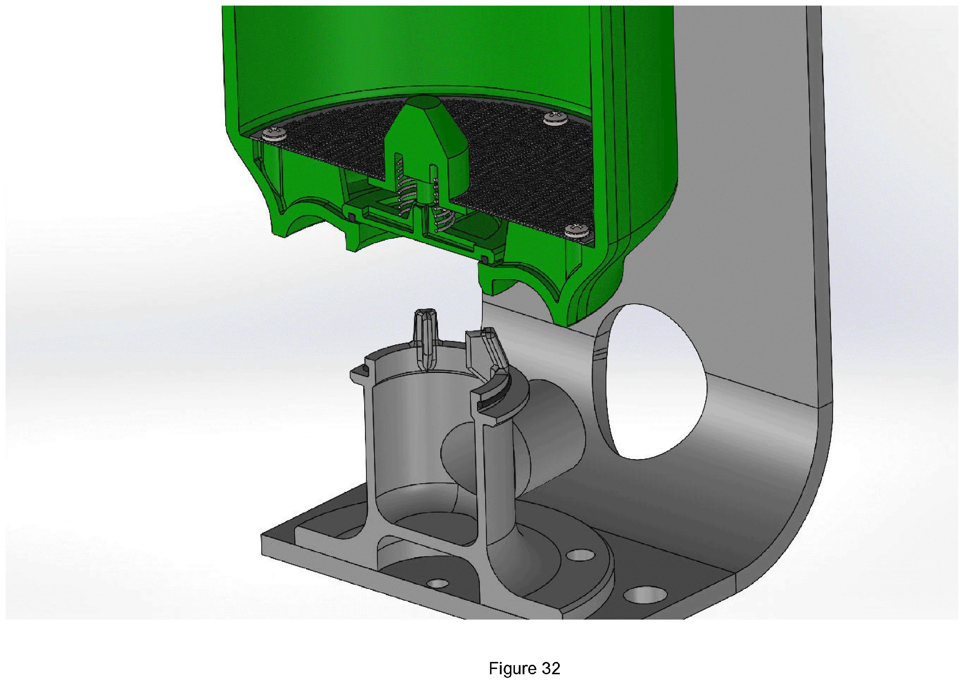

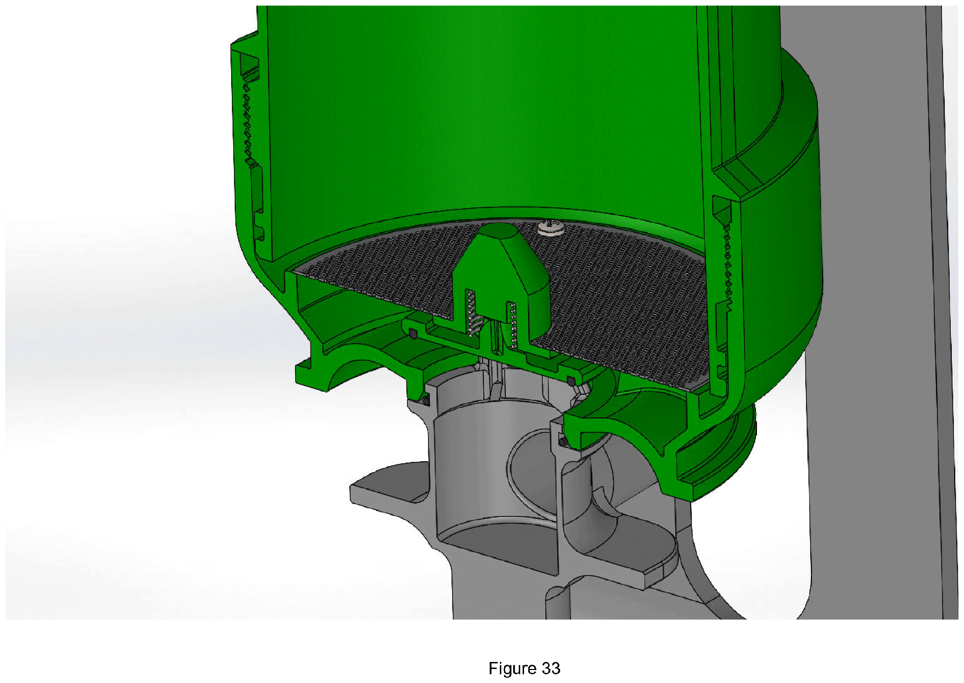

Figure 29 shows the canister 350 of Figures 22 and 23 being moved in the mount 352. Figure 30 is a section of Figure 29. Upper and lower canister actuators are provided by the mount. The upper and/or lower canister actuators may be spring loaded to facilitate entry/insertion of the canister. In this embodiment the upper actuator can be lifted "up" - see Figures 29 and 30 - and then released "down" - see Figure 31. This would move the lower end of the body down onto the lower actuator (see Figures 32 and 33).

Movement of the canister 350 into the mount 352 causes upper and lower valves 370 at the ends of the body to be opened. The valves may be described as inlet and outlet valves, depending on which direction air is flowing through the body. Similarly the inlet/outlet of the body may be described as through flow openings e.g. upper and lower openings/apertures formed at or towards each end of the body.

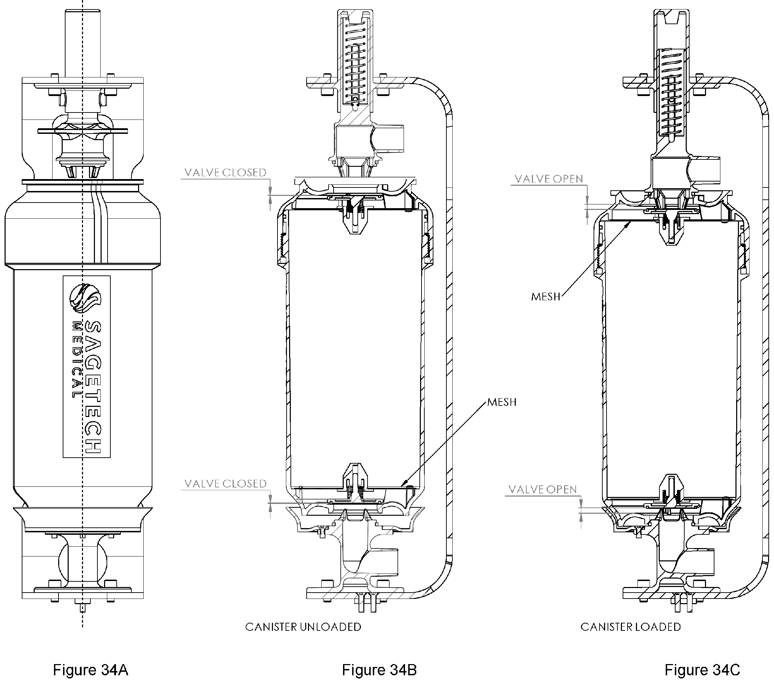

Figures 34A to 34C illustrate a capture canister in unloaded and loaded configurations.

The valves are supported by mesh plates (which act as diffusers). The part of the valve which blocks/unblocks the final outlet at either end of the body may be spring loaded and move up and down in a valve carrier which itself is supported in the mesh. The moving part of the valve may be provided with a sealing gasket to seal against the respective body opening.

The actuators from the mount move partially into the body openings to provide a sealed gas flow path through the body in use (see Figures 31 and 33). In this embodiment the actuators form part of and link into the dock.

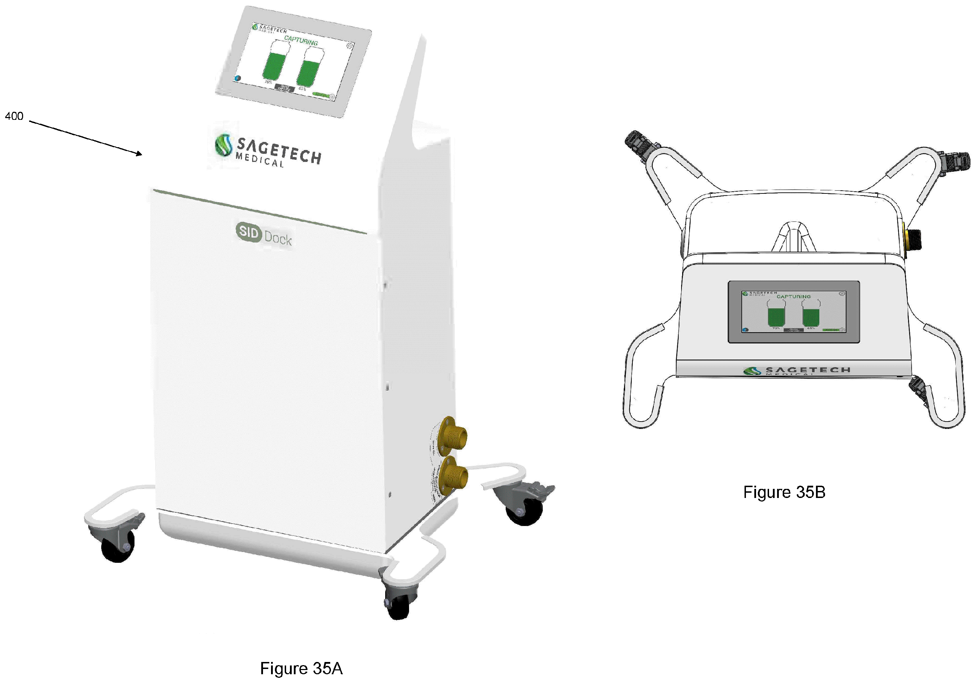

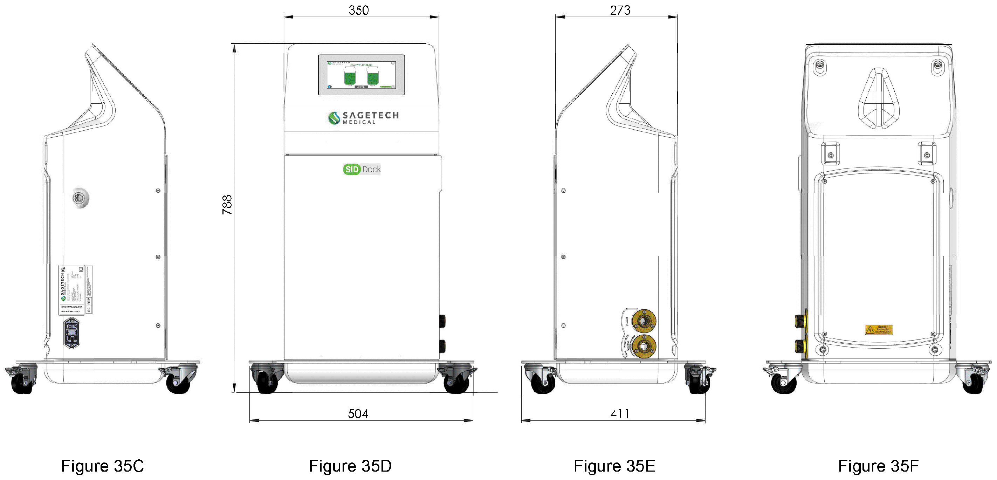

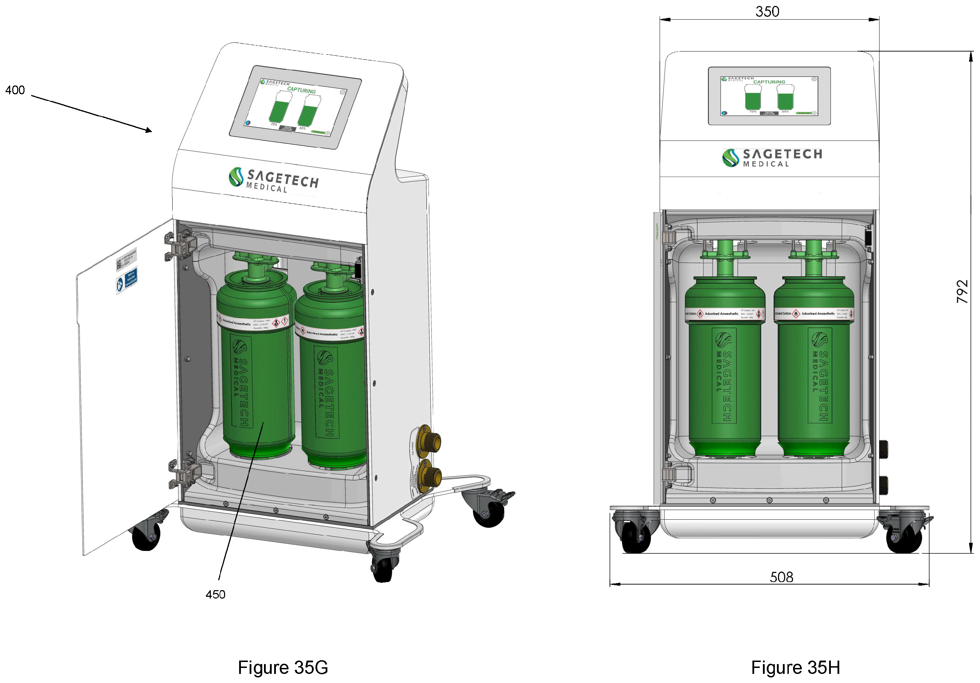

Figures 35A to 35H illustrate a dock 400 formed according to an embodiment of the present invention. Dimensions shown are illustrative and non-limiting.

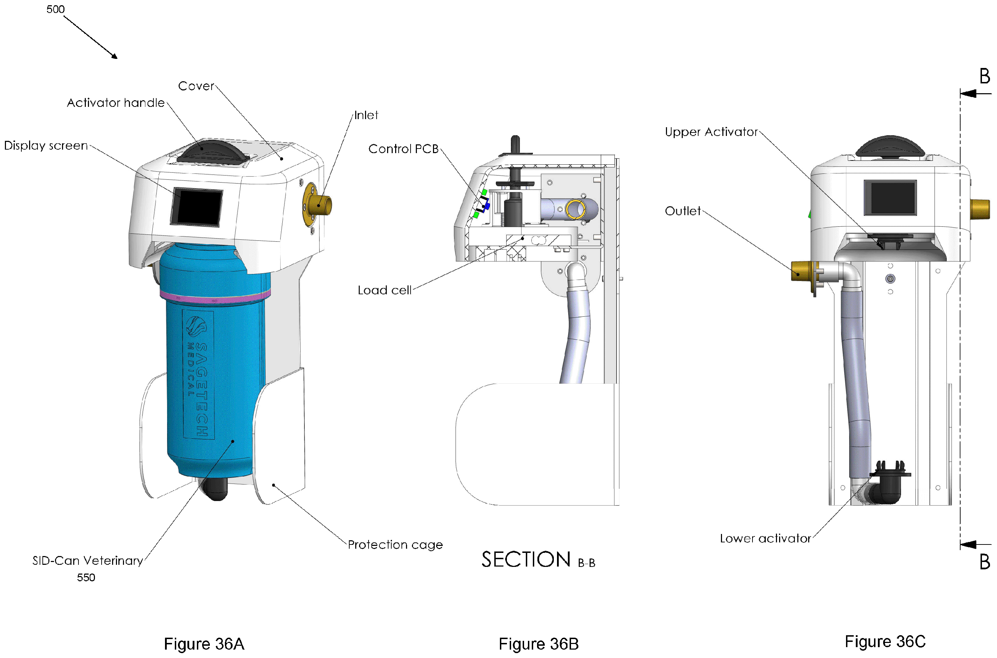

Figure 36A to 36C show a dock device 500 formed according to a further embodiment.

The dock 500 includes an upper activator and a lower activator for receiving and activating a can 550.

The dock 500 includes a manual activator handle. The handle is lifted to allow a can to be introduced, then forced down to make the can ready for use.

As part of the control system the dock can be placed in a capture mode or a bypass mode.

In other embodiments a sensor may be provided to detect changes in the gas stream and determine an operation mode.

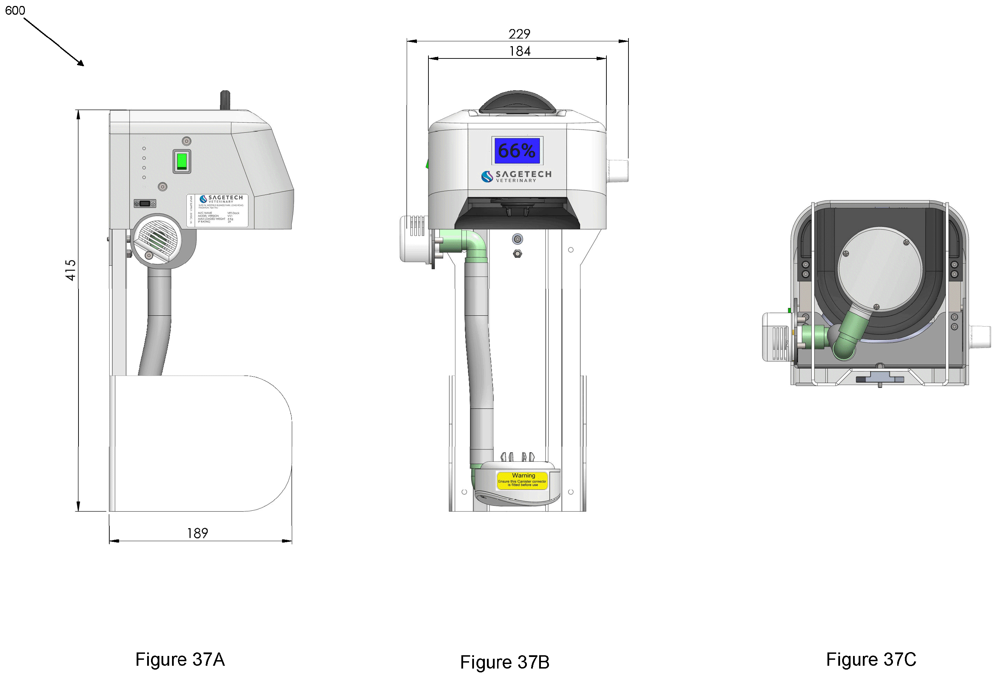

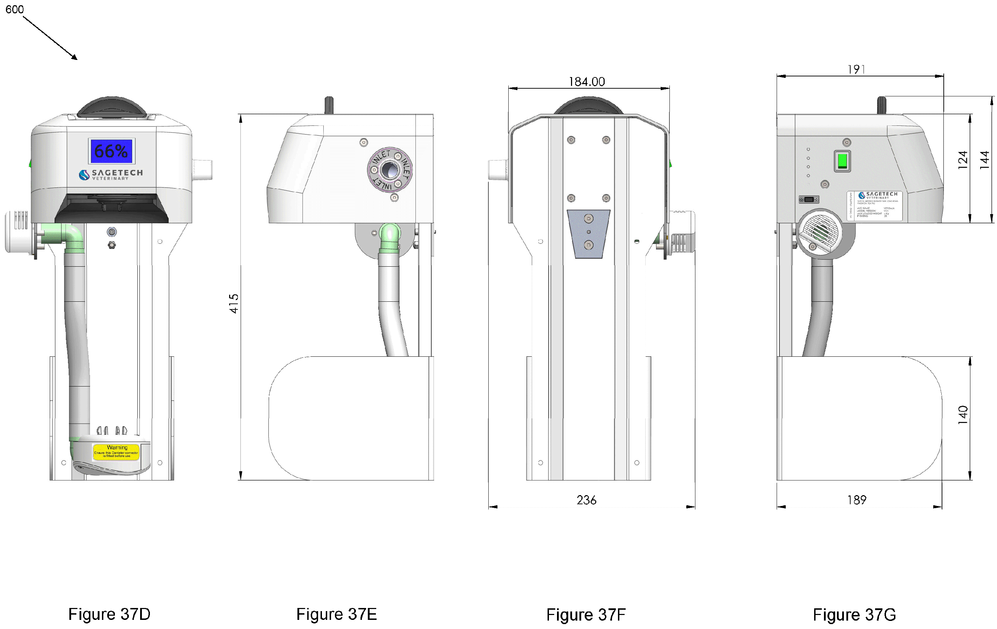

Figures 37A to 37G show a dock 600 formed according to a further embodiment.

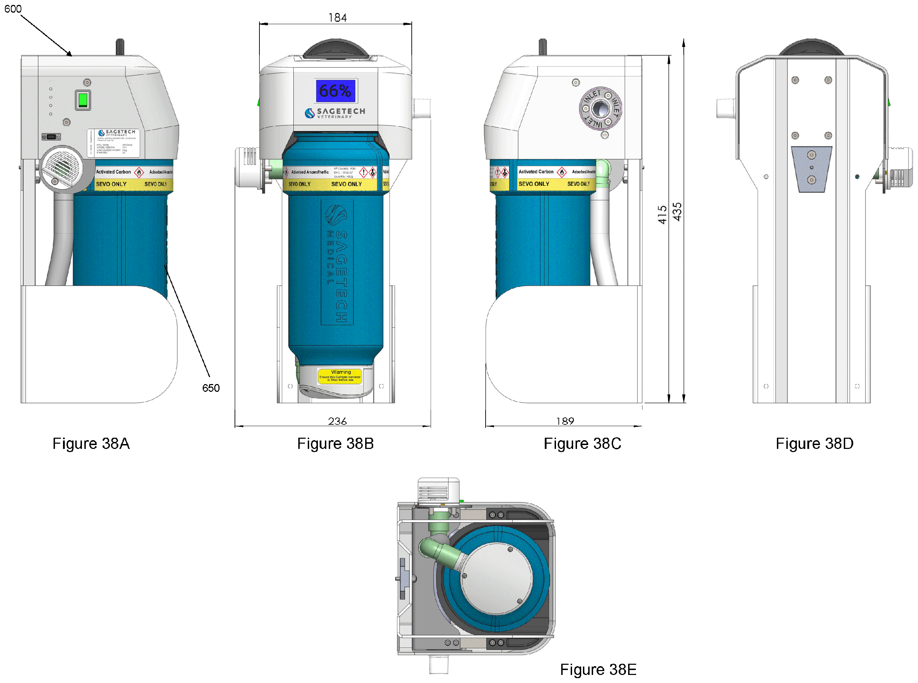

Figures 38A to 38E show the dock 600 with a can 650 loaded.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention.

## Claims

1. An anaesthetic gas capture device for extracting anaesthetic agent from a gas flow, the device comprises or can be connected to suction means for drawing gas flow to the device, the device is configured to receive one or more capture canisters through which gas can flow, the suction means operates upstream of the capture canister/s whereupon the suction means pushes gas through the or one or more of the canisters under positive pressure and agent extraction occurs under positive pressure, **characterised in that** the or each canister comprises a body for receiving filter material and through which gas containing anaesthetic agent can flow, the canister includes an inlet and an outlet, a valve is provided for the inlet and the outlet, the inlet and outlet valves being configured to be normally closed to seal the body and configured to open when the canister is loaded into the anaesthetic gas capture device.

2. A device as claimed in claim 1, in which the device is configured to draw gas from an anaesthetic machine, or in which the device is configured to draw from a patient, or in which the device is configured to draw from a patient mask.

3. A device as claimed in any preceding claim, in which the device comprises a sensor for sensing the presence of anaesthetic agent in gas flow.

4. A device as claimed in claim 3, in which the sensor is provided on an inlet side of the device,

5. A device as claimed in claim 3 or claim 4, in which the suction means is provided upstream of the capture canister/s and downstream of the sensor,

6. A device as claimed in any of claims 3 to 5, in which the sensor can activate the suction means when anaesthetic agent is detected.

7. A device as claimed in any of claims 3 to 6, in which the sensor activates and/or deactivates the suction means based on detection of a threshold agent concentration or amount.

8. A device as claimed in any preceding claim, having a capture mode and a bypass mode.

9. A capture canister for a device according to any preceding claim.

10. A capture canister for an anaesthetic gas capture device, the canister comprises a body for receiving filter material and through which gas containing anaesthetic agent can flow, the canister includes an inlet and an outlet, a valve is provided for the inlet and the outlet, the inlet and outlet valves being configured to be normally closed to seal the body and configured to open when the canister is loaded into an anaesthetic gas capture device.

11. A canister as claimed in claim 10, in which the inlet valve and/or outlet valve include a return spring.

12. A canister as claimed in claim 10 or claim 11, in which the inlet valve and/or outlet valve is formed as a poppet valve.

13. A canister as claimed in any of claims 10 to 12, in which the canister comprises a mesh plate at or towards the inlet and/or outlet.

14. A canister as claimed in claim 13, in which the mesh plate/s act as a diffuser.

15. A canister as claimed in claim 13 or claim 14, in which part of the valve is positioned in and/or supported by the mesh.
